# EUROPEAN PATENT SPECIFICATION

(11) **EP 2 839 359 B1**
(45) Date of publication and mention of the grant of the patent: **18.12.2019**
(21) Application number: 13778247.0
(22) Date of filing: 18.04.2013
(51) Int. Cl.: G16H 40/63, G16H 20/30

(54) **SMARTPHONE-CONTROLLED ACTIVE CONFIGURATION OF FOOTWEAR INCLUDING WITH CONCAVELY ROUNDED SOLES**
SMARTPHONE-GESTEUERTE AKTIVE KONFIGURATION EINES SCHUHWERKS MIT KONKAV ABGERUNDETEN SOHLEN
CONFIGURATION ACTIVE, COMMANDÉE PAR TÉLÉPHONE INTELLIGENT, DE CHAUSSURES Y COMPRIS AVEC SEMELLES ARRONDIES DE FAÇON CONCAVE

(30) Priority: 18.04.2012 US 201261687072 P; 19.04.2012 US 201261687127 P; 11.03.2013 US 201361851598 P; 14.03.2013 US 201361851869 P; 15.03.2013 US 201361852038 P; 10.04.2013 US 201313859859; 11.04.2013 US 201313861207
(43) Date of publication of application: 25.02.2015
(73) Proprietor: Ellis, Frampton E., Jasper, FL 32052 (US)
(72) Inventor: Ellis, Frampton E., Jasper, FL 32052 (US)
(74) Representative: De Vries & Metman
(86) International application number: PCT/US2013/037045
(87) International publication number: WO 2013/158809

(56) References cited:
- KR-A- 20090 049 572
- KR-A- 20110 071 727
- US-A1- 2007 006 489
- US-A1- 2008 086 916
- US-A1- 2011 056 093
- US-A1- 2011 197 157

## Description

### BACKGROUND

In many prior U.S. Patents, including for example both U.S. Patent no. 5,317,819 and U.S. Patent no. 6,163,982, the applicant has shown in detail the inherent stability defects in most modern footwear, which are structurally flat instead of wrapping around the anatomically rounded shape of an intended wearer's foot sole, as required in order to preserve the naturally superior biomechanical stability of the intended wearer's bare foot sole.

However, there is also high degree of complexity inherent in correctly designing and manufacturing anatomically neutral footwear due to the extremely complex structure of the human foot. The result is that nearly all commercially available footwear available currently significantly degrade the natural stability of the barefoot, resulting in needless chronic and acute injuries.

But the alternative of bare feet alone is not the answer, since bare feet are often unsuited for the modern environment, since they fail to provide insulation against extreme heat or cold, protection against sharp objects or dangerous chemicals, and traction on artificial sports or other unnatural surfaces.

With no practical alternatives, a wearer of modern footwear is forced into a lifetime of defective footwear use that all too frequently results in anatomical structure and gait problems that cause severe acute and chronic injury to joints and other health issues. Unfortunately, with existing technology, only the symptoms of the injury are ever treated, because there is currently no way to easily evaluate and identify the underlying specific footwear causes of the injury or to eliminate those causes or reduce their severity.

Nor is there a way to provide immediate and effective testing and evaluation to find the most optimal footwear solution as quickly as possible. Nor is there a way then to immediately implement that most optimal footwear solution, while afterwards continuing indefinitely the ongoing testing and evaluation to prevent future problems. Nor is there as way to share these individual optimal solutions among larger population groups to achieve potentially many other tangible health benefits among similar subgroups.

US 2007/0006489 discloses footwear that includes configurable structures and sensors, both located in the footwear. A control system is provided to receive and process data from the sensors in the footwear and control the configuration of one or more of the configurable structures based on the processed data. The footwear may include a variety of different types of sensors including an accelerometer and/or gyro sensors.

### SUMMARY

In a first aspect, the invention relates to a smartphone or other mobile computer device as claimed in claim 1. In another aspect the invention relates to a sole or a removable sole insert of footwear of a wearer as claimed in claim 21. In yet another aspect, the invention relates to a computer program as claimed in claim 22.

In some aspects, the applicant's new footwear soles emphasize an extraordinarily simple approach, which is simply bending the sides of footwear sole up in the direction of the foot, instead of leaving the sole structure conventionally flat like the ground. The result is a new footwear sole that is simply concavely rounded underneath the intended wearer's foot sole, particularly as viewed in frontal plane cross-sections.

The new footwear soles are not complex to design and manufacture, since they avoid dealing with the enormous complexity of trying to conform to the irregularly shaped human foot structure. They still, however, preserve most if not all of the essential biomechanical superiority of the barefoot in natural pronation and supination motion when load bearing on the ground, even when shod with any of the variations of the new sole described in this application -- as long as the footwear sole's concave rounding is configured to deform under a body weight load to flatten against the flat ground, as does a barefoot sole.

The applicant's inventions also include using a smartphone device with motion sensors and/or in-shoe force and/or pressure sensors to easily evaluate and identify the underlying specific footwear causes of a footwear injury and to eliminate those causes and to eliminate or reduce the severity of their impact on the wearer's body, using the applicant's inventions that also include footwear with bladders, compartments, chambers and/or sipes configured to be controlled by the smartphone in real time and can also include the concavely rounded sole structure invention described above and elsewhere in this application.

With the applicant's smartphone and configurable footwear, there a way to provide immediate and effective testing and evaluation to find the most optimal footwear solution as quickly as possible. In addition, with them there is a way then to immediately implement that most optimal footwear solution, while afterwards continuing indefinitely the ongoing testing and evaluation to prevent future problems. Furthermore, with them there is a way to share these individual optimal solutions among larger population groups to achieve potentially many other tangible health benefits among similar subgroups.

### BRIEF DESCRIPTION OF THE DRAWINGS

Figure 1A and 1B are examples of smartphones, the iPhone 5™ and the Samsung Galaxy S III™, respectively, which can be used to actively configure footwear and other apparatus.
Figure 2A and 2B are example ear phones and headphones, respectively, which can include motion, proximity, and other sensors 582.
Figure 3 shows side and back views of a human skeleton to illustrate some of the potential positions for the smartphone and for sensors on the body or apparel or equipment, including examples of footwear, pants, belt, collar, wristband, earphones, and helmet.

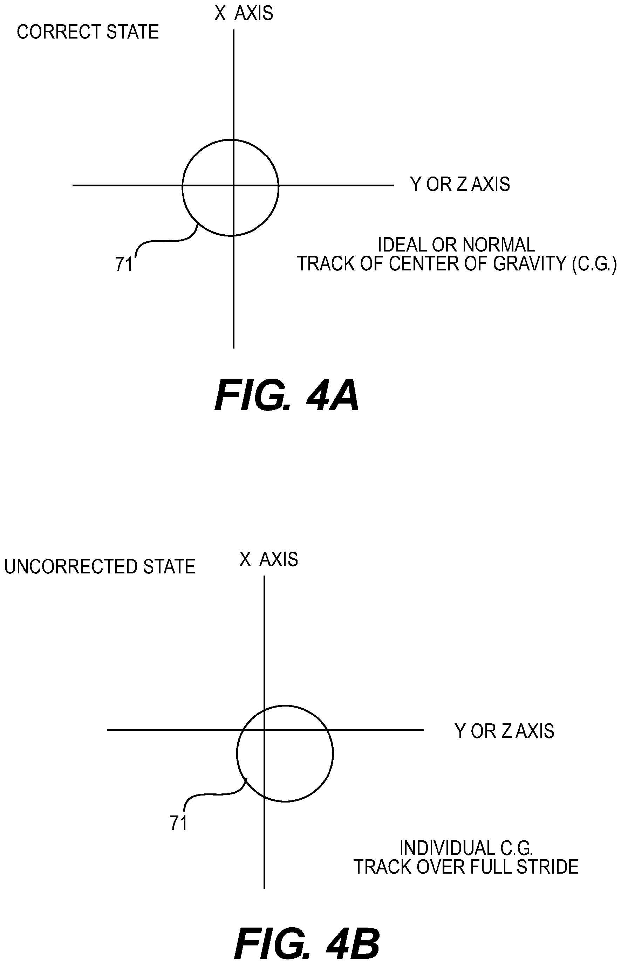

Figure 4A and 4B show two axis graphs showing examples of tracks of center of gravity (C.G.) motion over at least a full locomotion stride, Fig. 4A showing a model or correct or preferred state and Fig. 4B showing uncorrected or misaligned state.

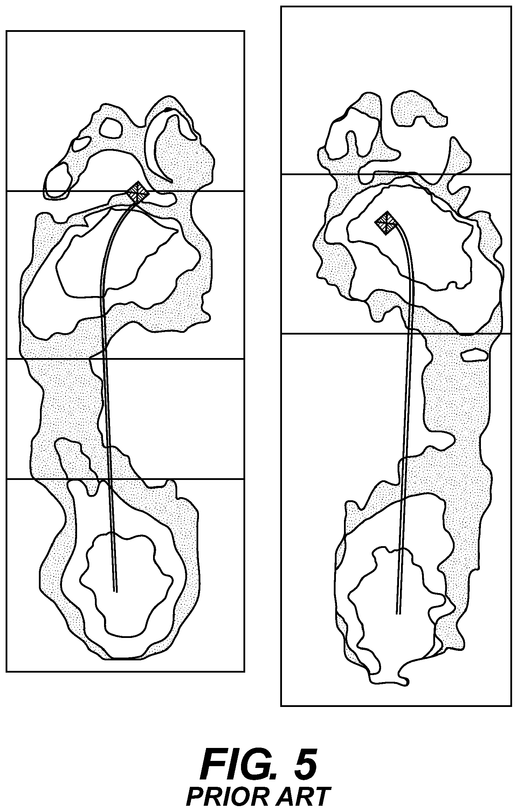

Figure 5 shows a prior art example of the left and right foot force and relative pressure measurements provided by an existing in-shoe system by F-Scan™.

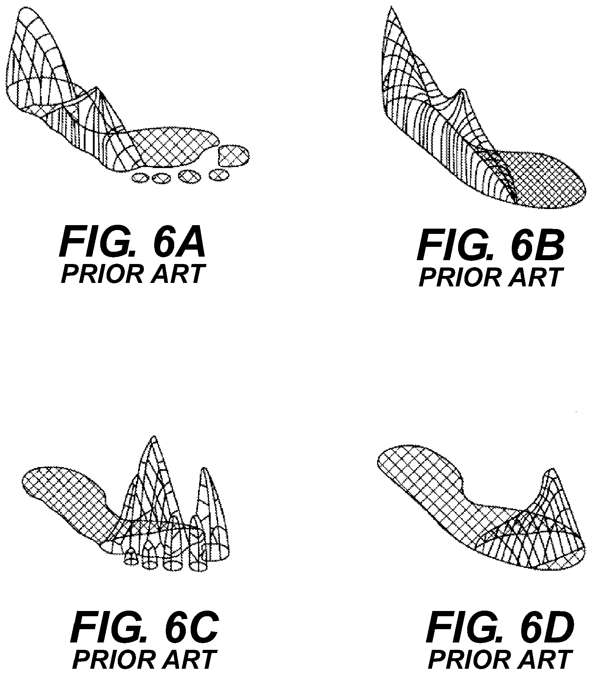

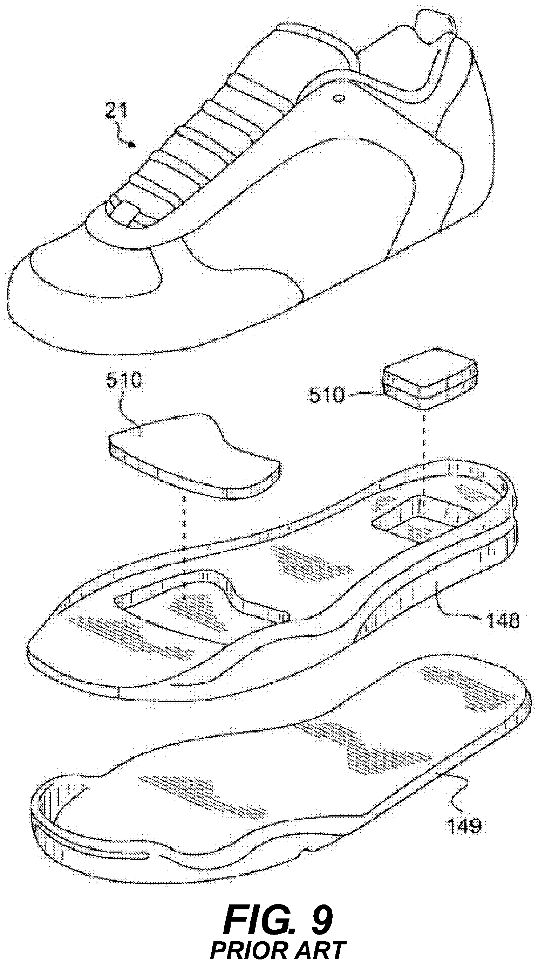

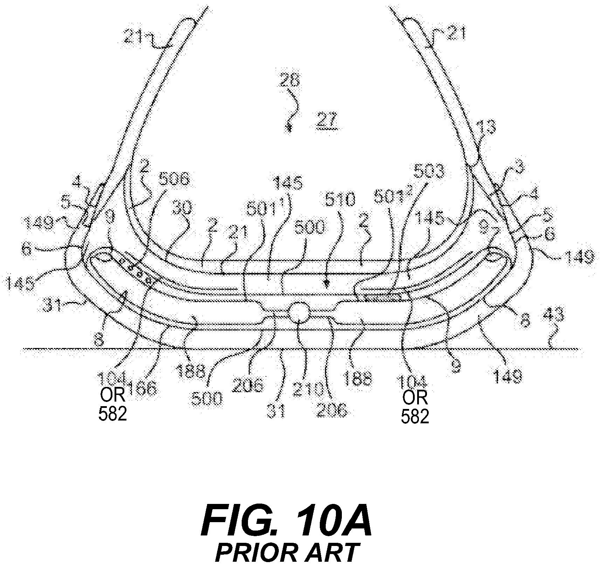

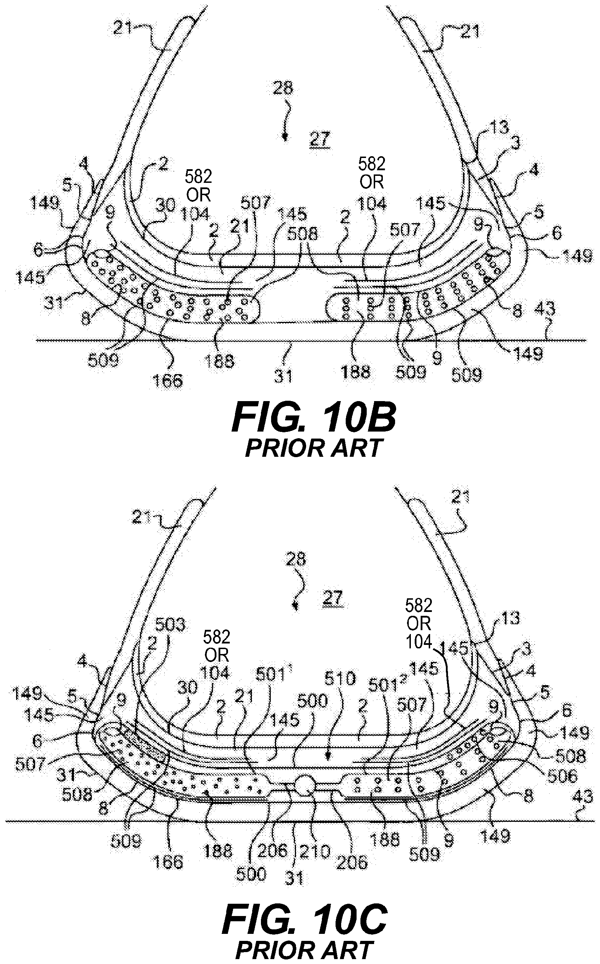

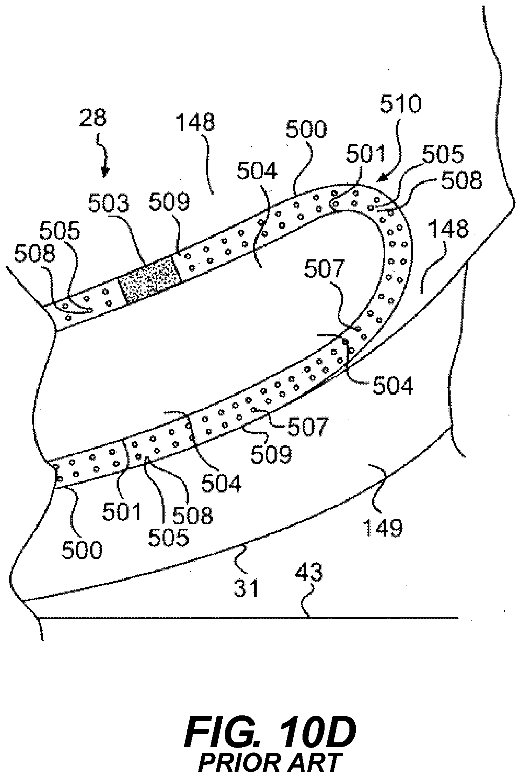

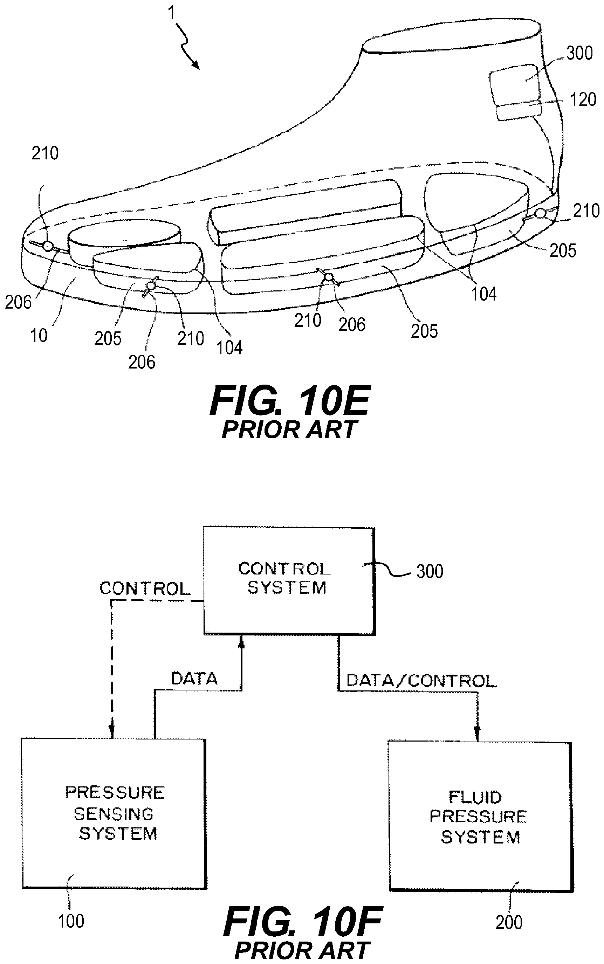

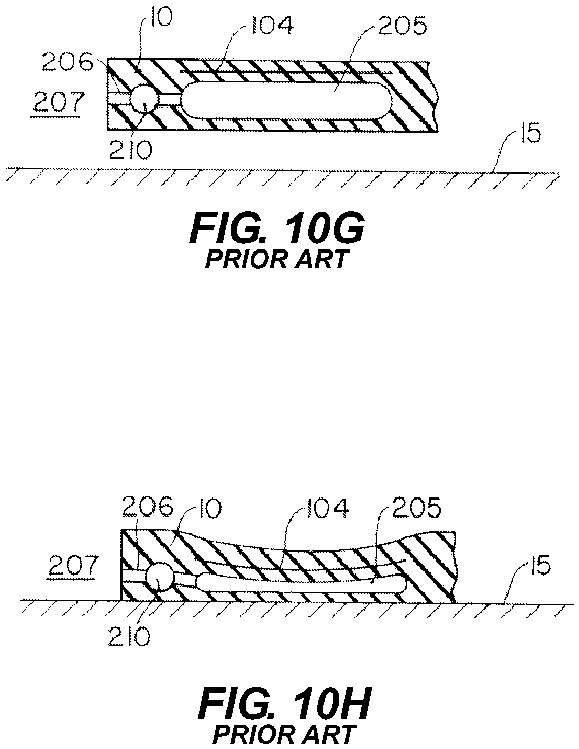

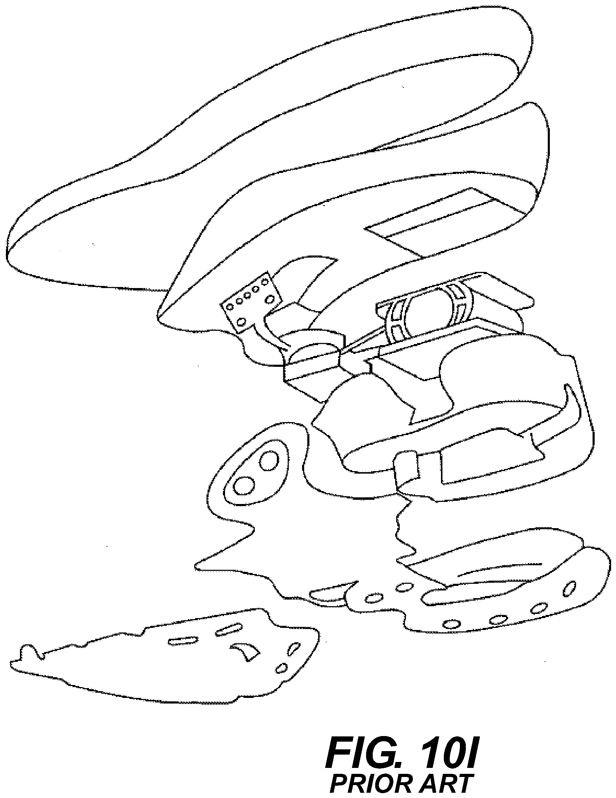

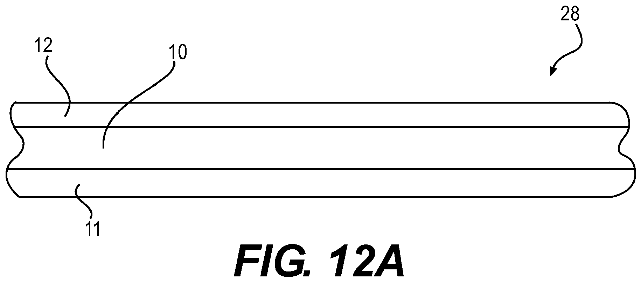

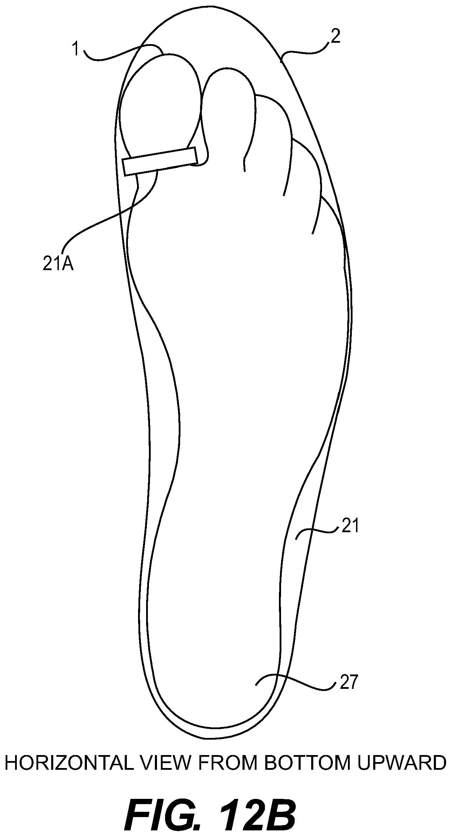

Figures 6A-6D are other prior art examples showing insole pressure measurements of the running stride and are Figures 9-12 of the applicant's U.S Patent no. 5,909,948.

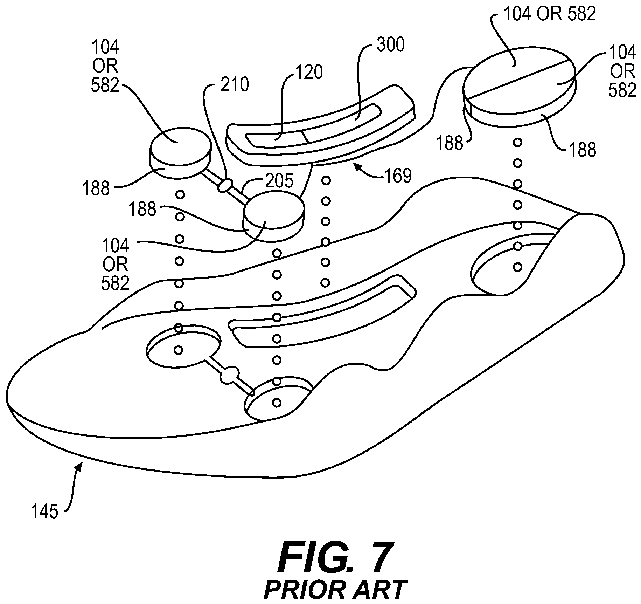

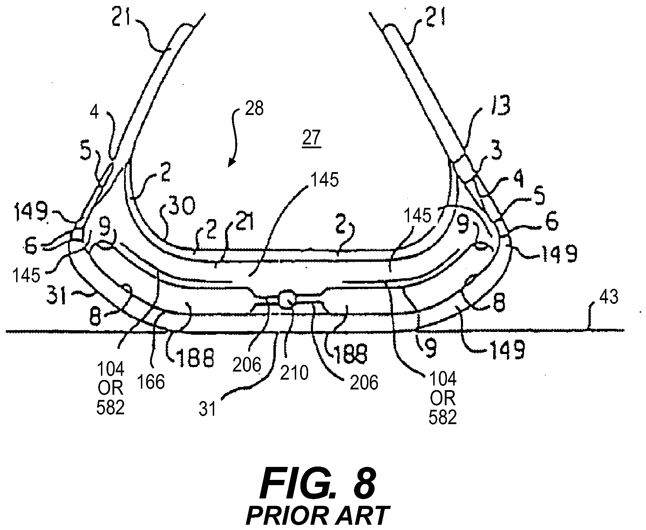

Figure 7 shows a perspective view of a prior art example of footwear with computer controlled bladders, compartments, or chambers located in a removable midsole section and was Figure 11P of the applicant's US 2006/0248749 A1 (the "'749 publication"). Figure 8 shows a frontal plane view of a similar embodiment and was Figure 11N of the same '749 publication.
Figure 9 shows a perspective view of a prior art example of footwear with inner bladders, compartments, or chambers of Figure 7 & 8 with internal flexibility sipes and outer, bladders, compartments, or chambers and was Figure 1C of the applicant's US 2008/0086916 A1 (the "'916 publication").
Figure 10A-10D are Figures 15, 16, and 17A-17B of the applicant's '916 publication and show footwear prior art computer controlled inner bladders, compartments, or chambers surrounded by internal flexibility sipes and outer bladders, compartments, or chambers. Figures 10E-10H show prior art examples Figures 1, 2, 4A and 4B from Demon's U.S. Patent No. 5,813,142 of computer controlled valves venting from bladders to outside the shoe sole. Figure 10I is Figure 44 of the same publication and shows a footwear prior art computer controlled mechanical cushioning system.
Figure 11A-11I are examples of the applicant's semi-thong inventions. Figure 11A shows a horizontal view of the upper surface of a footwear sole with an intended wearer's footprint superimposed in its normal position with the semi-thong 3 located between the big and second toes; and optional other semi-thong locations indicated between the other toes. Figures 11B-11E show horizontal cross-sections of the semi-thong; Figure 11F shows a frontal plane cross-section showing the location of an example semi-thong. Figures 11G-11I show examples of different semi-thong structures in cross-section.
Figure 12A is a blown up cross-section of an example part of the applicant's inventions of an extremely minimalist footwear sole or a traction sock or an individual thread. Figure 12B shows an example of the most minimalist of footwear, which is without any sole and only a big toe strap 21a (and/or other toe straps), elastic or other, to hold down the forefoot of the soleless footwear upper.

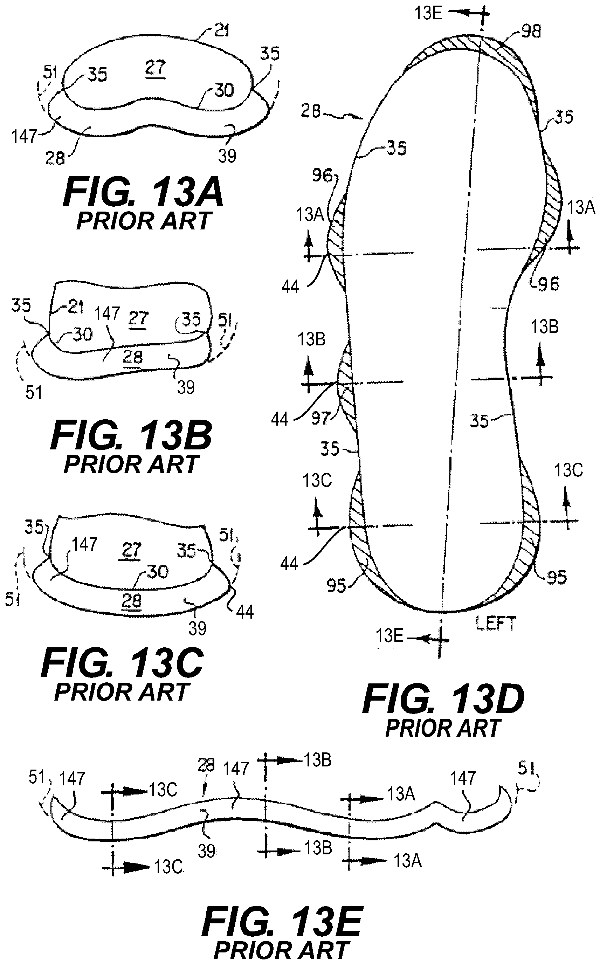

Figures 13A-13E is a prior art series showing the applicant's prior inventions of footwear soles conforming to the shape of an intended wearer's unloaded foot sole and was Figure 51A-51E of the applicant's U.S. Patent no. 7,334,350.

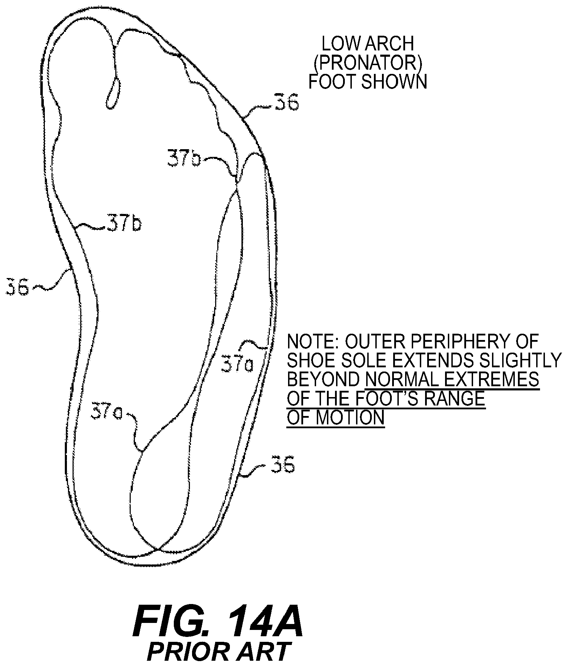

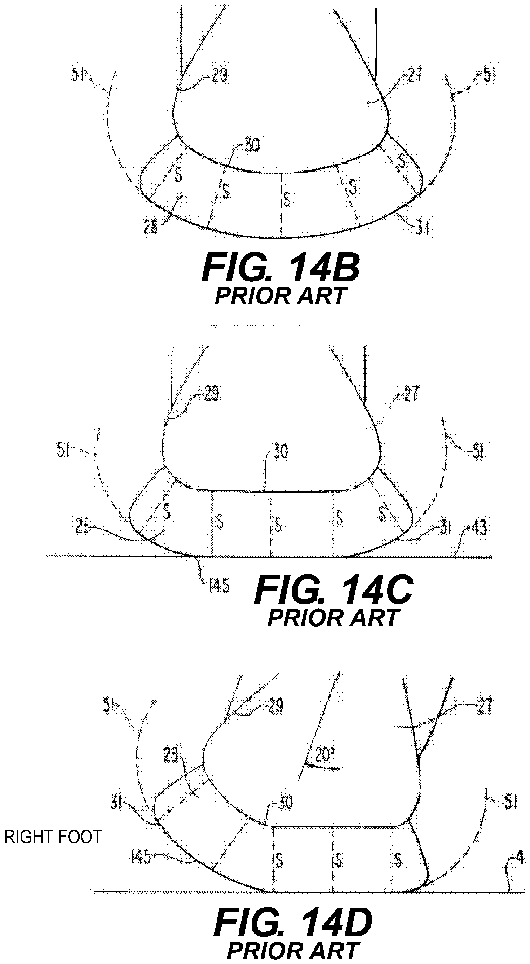

Figure 14A is prior art Figure 62 from the applicant's '350 U.S. Patent showing the extra sole width required to accommodate the dynamic footprint and Figures 14B-14D are prior art Figures 1A-1C from the applicant's U.S. Patent no. 6,163,982 showing the footwear sole's capability to deform to flatten under the body weight of a wearer, including during 20 degrees of supination or pronation.

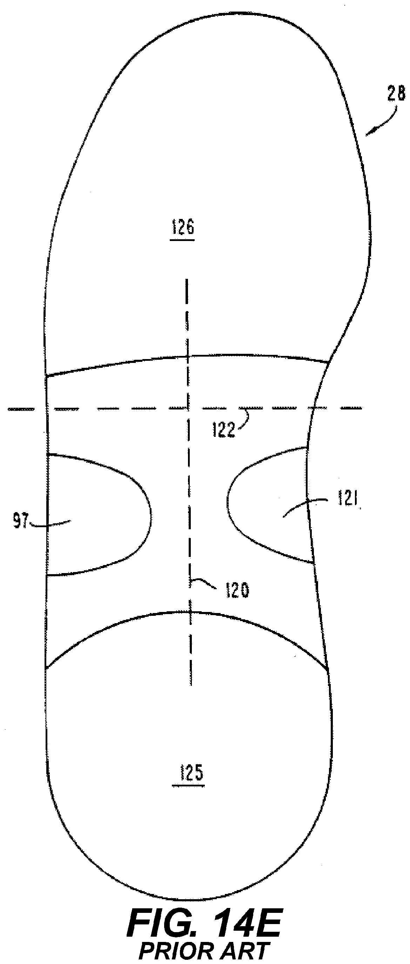

Figure 14E is the prior art Figure 28c of the applicant's U.S. Patent no. 5,317,819 showing the flexibility axis 122.
Figures 15A-15H is a series similar to Figures 13A-13E showing frontal plane and sagittal plane cross-sections and horizontal plan overview of the applicant's new inventions of a footwear sole that is concavely rounded relative to the intended wearer's foot sole in frontal plane cross-sections in the forefoot, midfoot, and heel areas of the footwear sole; Figs. 15E-15H show different potential variations that can be incorporated into the long axis structure shown in Figs. 15A-15D.

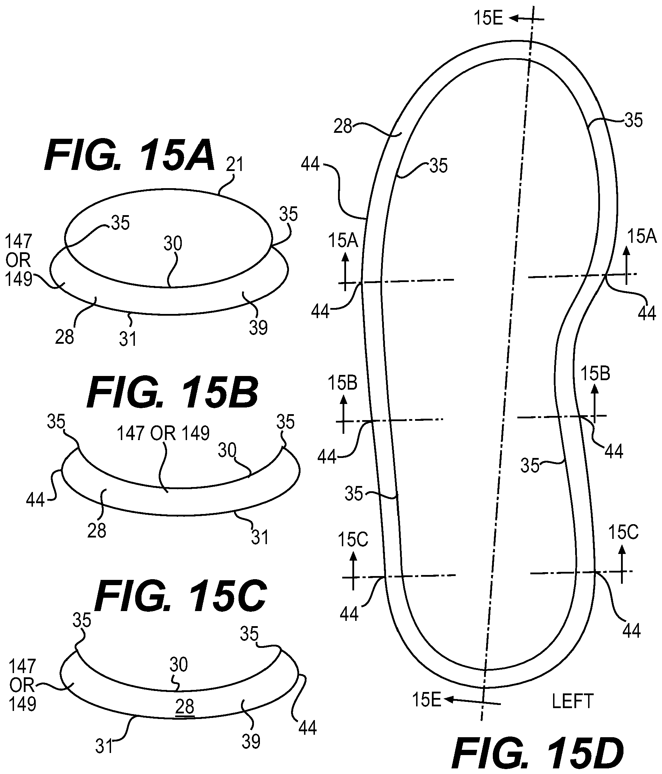

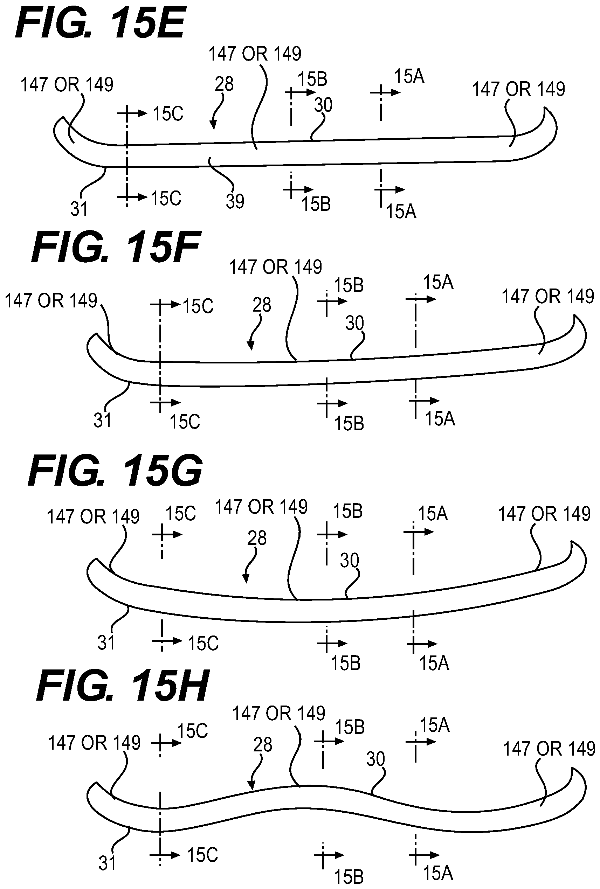

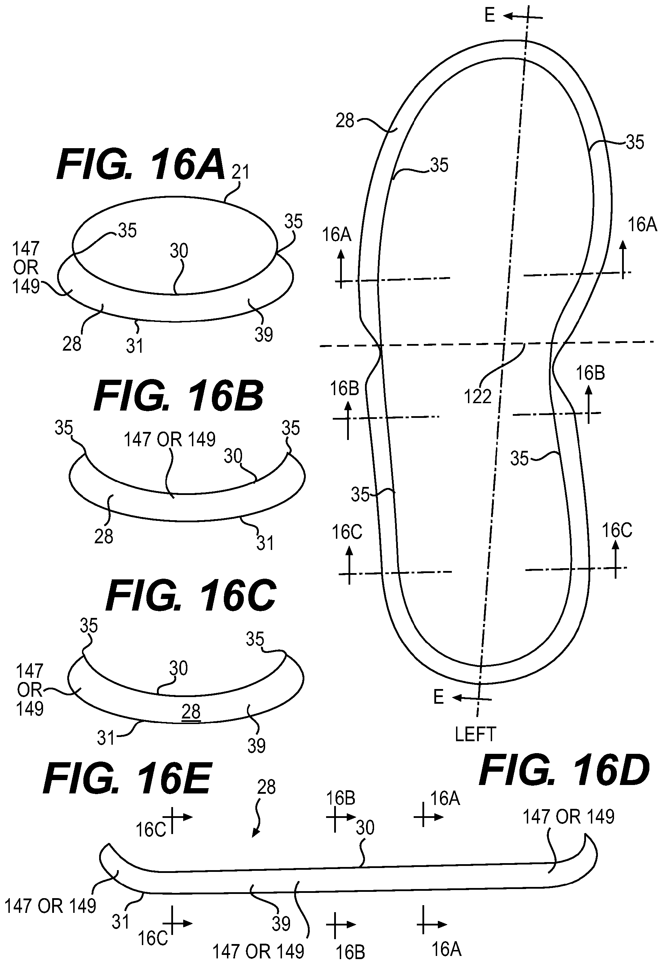

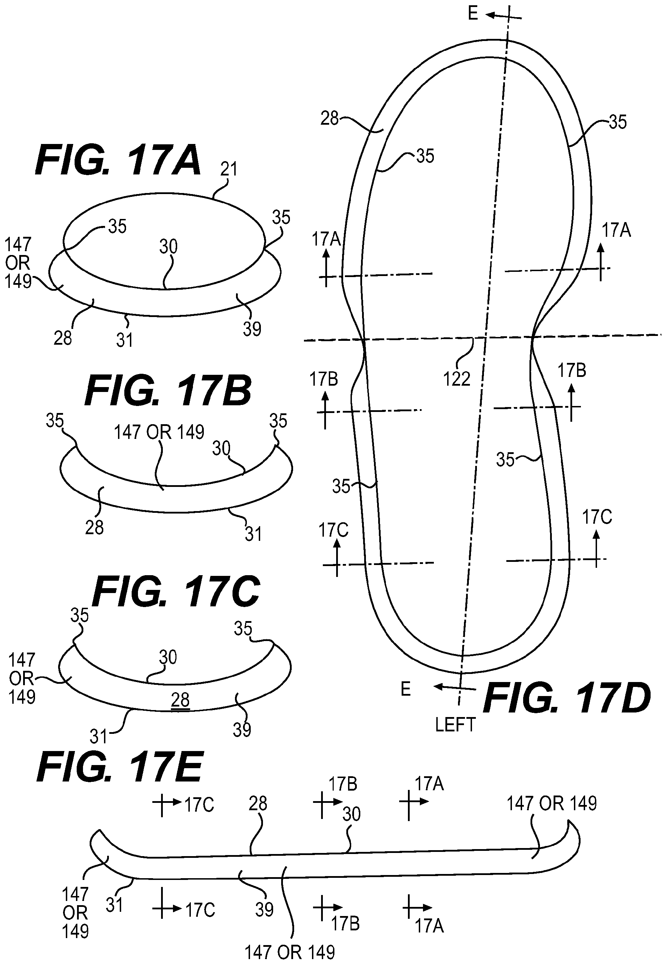

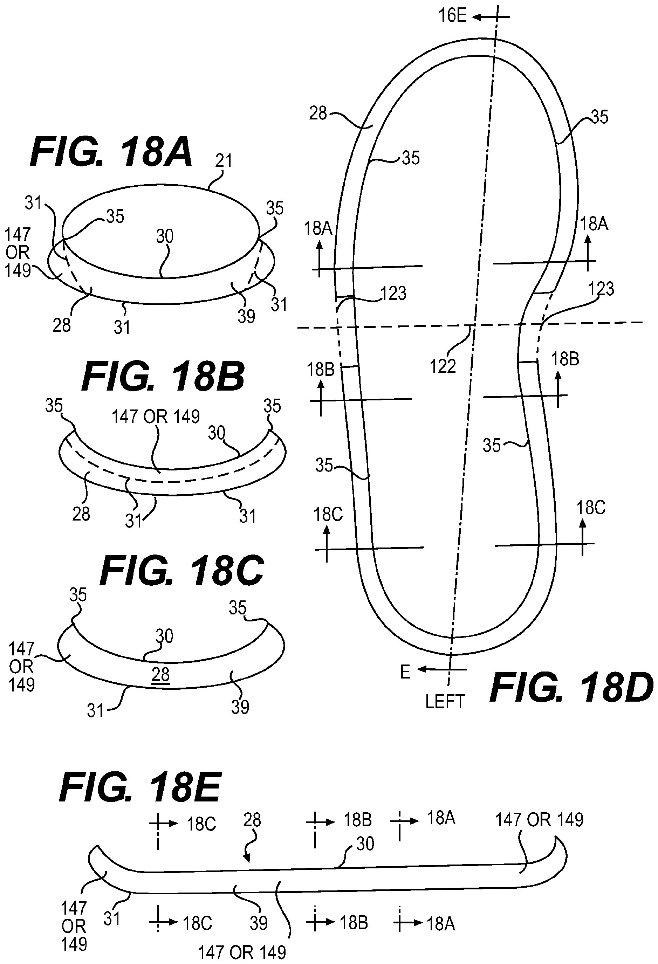

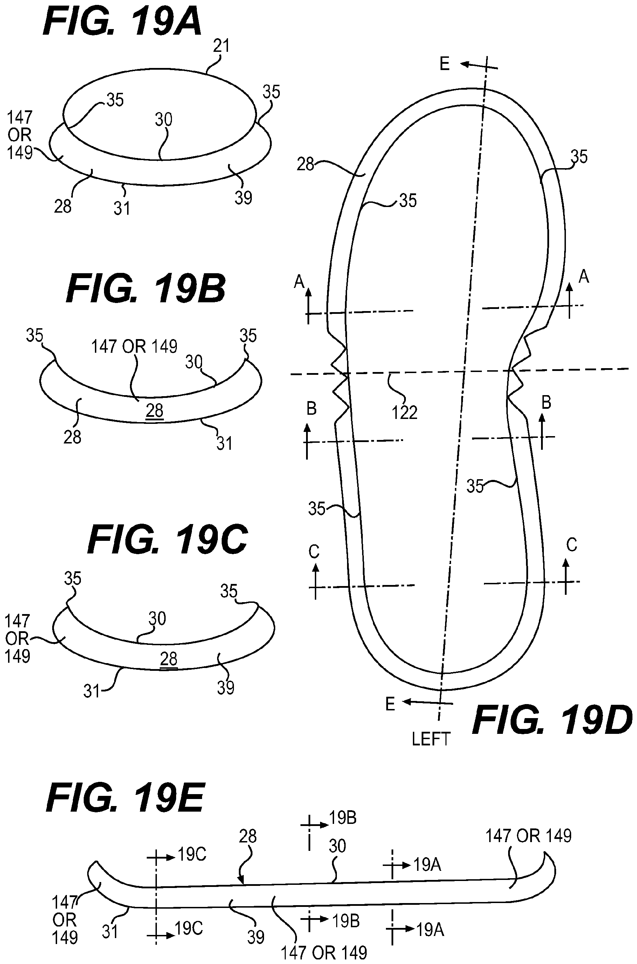

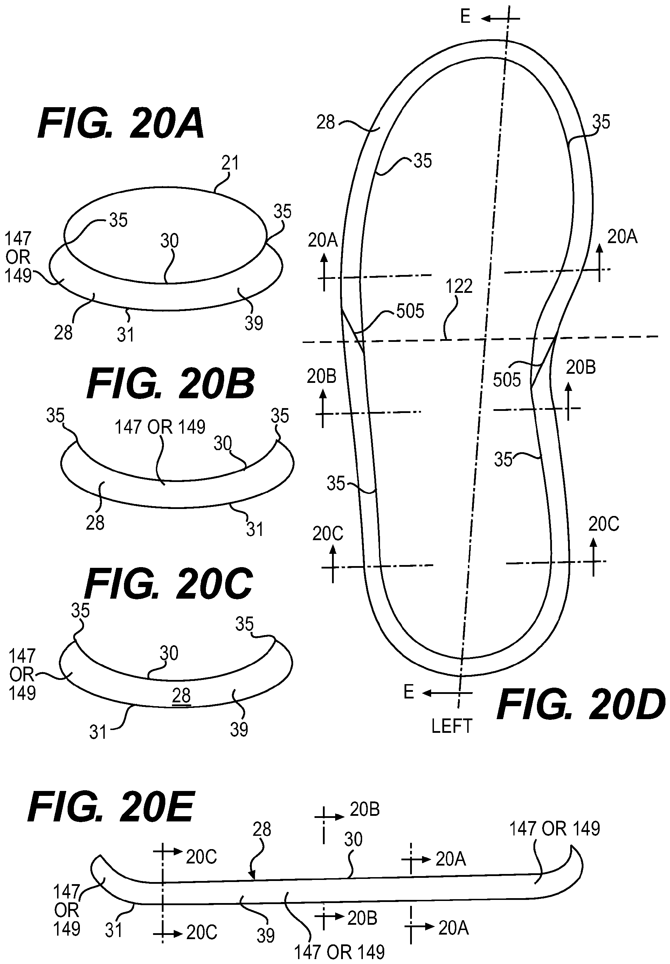

Figure 16A-16E is like Figure 15A-E, but with a flexibility groove just aft of the forefoot of the footwear sole on both sides, between the cross-sections of Figures 16A and 16B. Figures 17A-17E, Figures 18A-18E, Figures 19A-19E, and Figures 20A-20E show variations of the flexibility groove located proximate to flexibility axis 122, with Figure 18D also showing a fabric cover over the groove, the fabric cover optionally forming a portion of a strap for the upper and Figure 20D showing flexibility sipes 505 instead of grooves and Figures 18A and 18B showing with dashed line the position of the inset outersole 31 established by the groove.

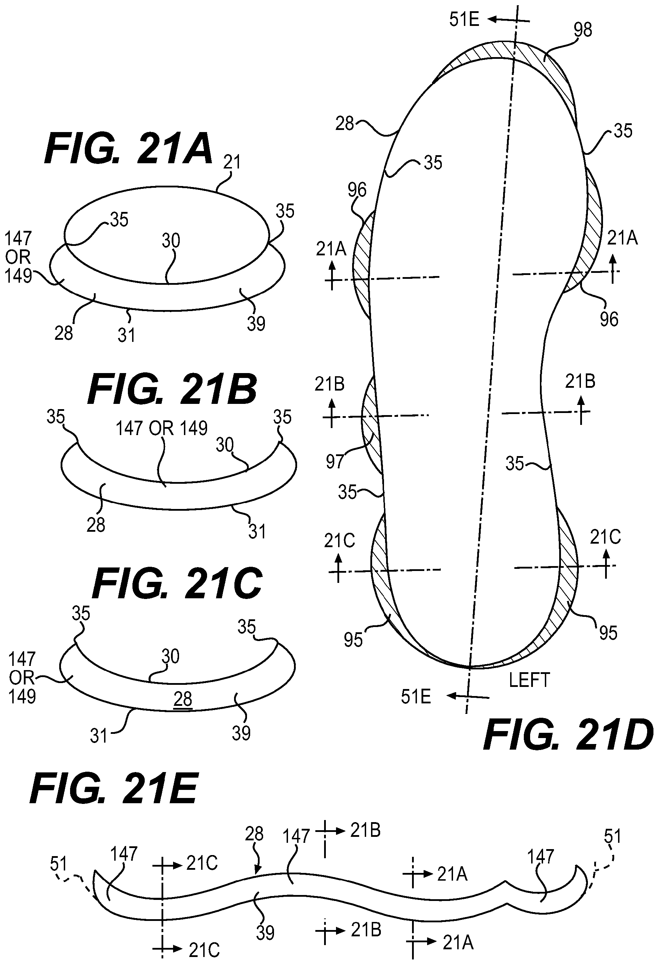

Figure 21A-21E is the applicant's concavely rounded footwear sole inventions applied to prior art Figures 13A-13E, which is an example embodiment with bulges and abbreviated sides for flexibility.

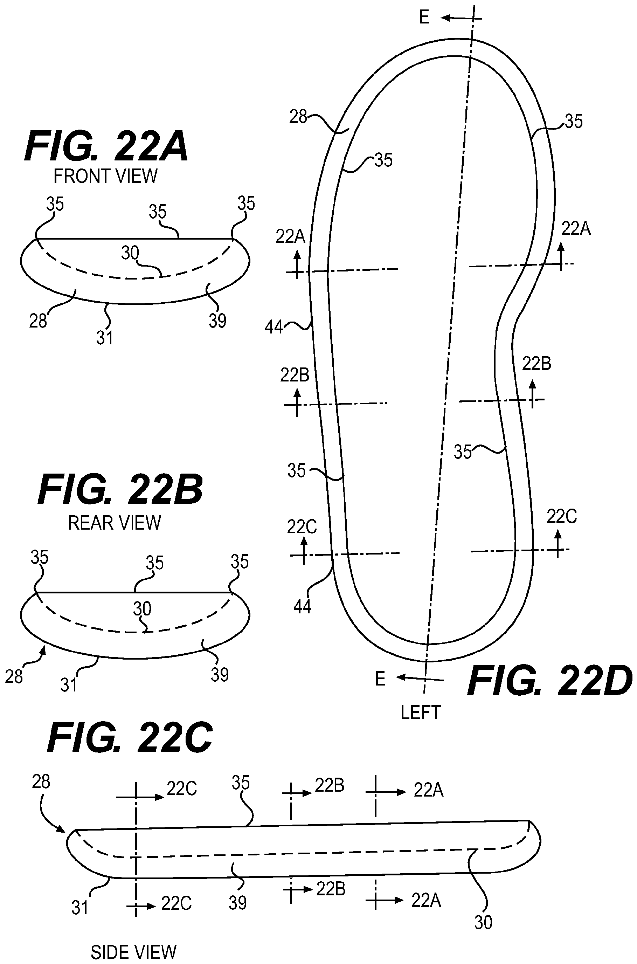

Figures 22A-22D are a front view, a back view, a side view, and an overhead view of the applicant's concavely rounded footwear sole shown in Figures 15A-15E, with inner dashed lines showing the inner surface 30 of the sole.
Figure 23A-23D are a front view, a back view, a side view, and an overhead view of the applicant's concavely rounded footwear sole shown in Figures 17A-17E, with inner dashed lines showing the inner surface 30 of the sole..

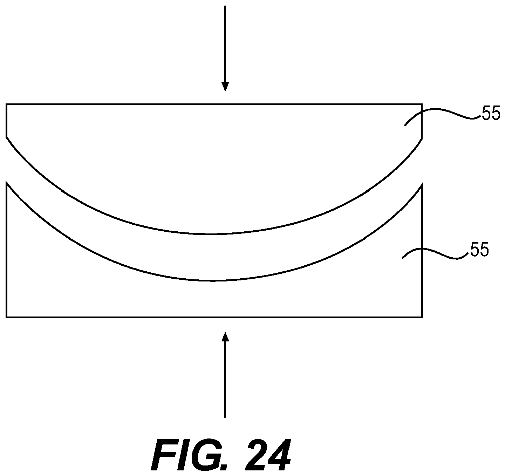

Figure 24 is a frontal plane cross-section of a press and/or press forms 55 configured structurally to form a concavely rounded footwear sole such as shown in Figures 15-23.

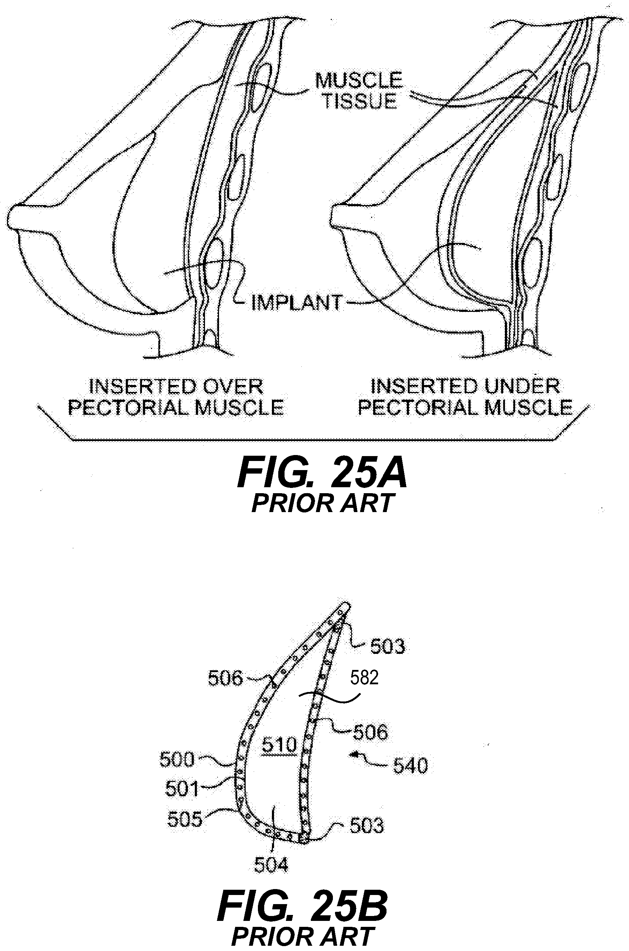

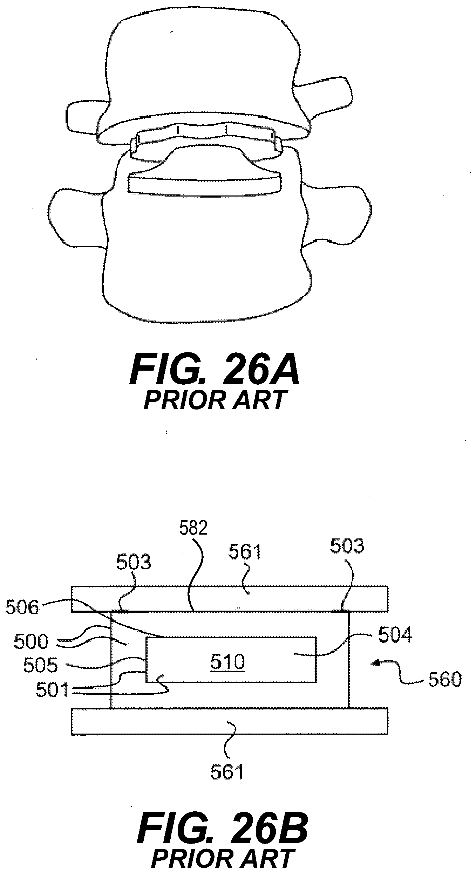

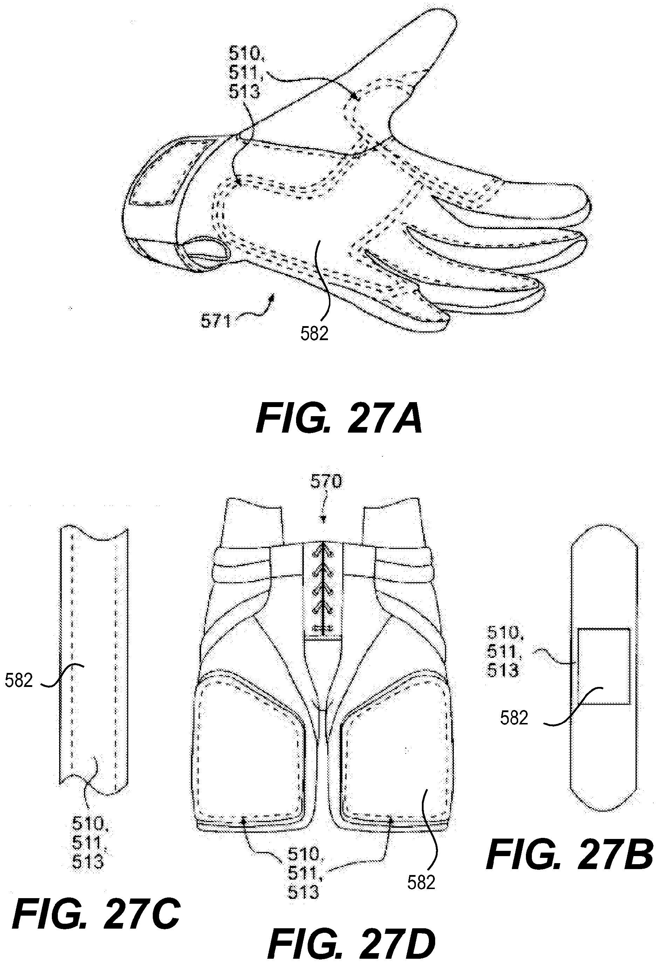

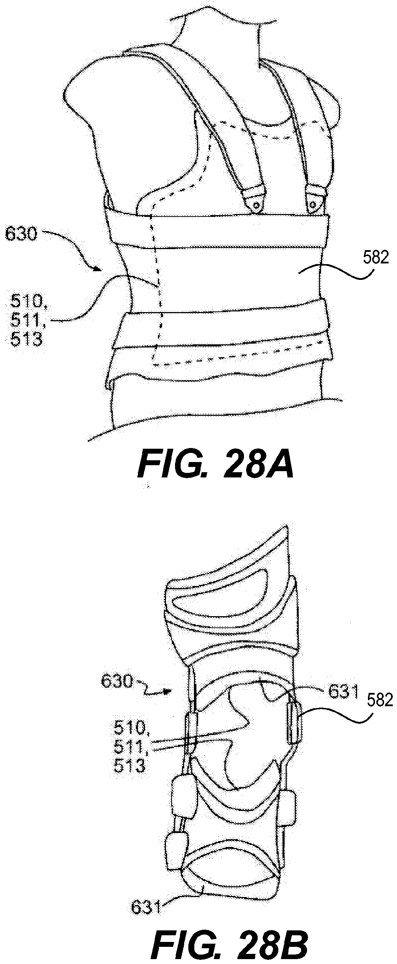

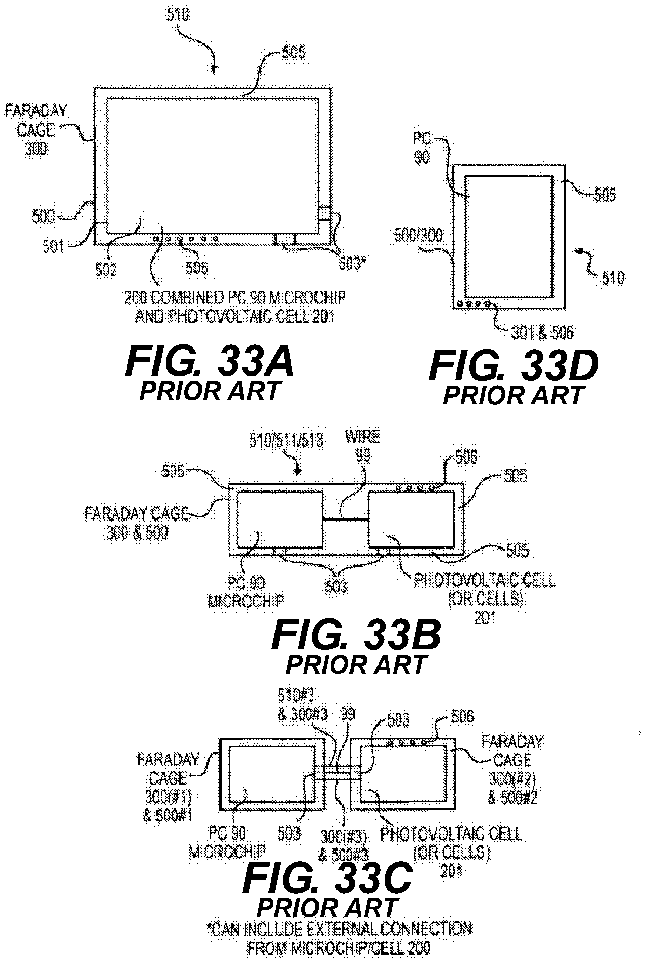

Figures 25B, 26B, 27A-27D, 28A-28B, 29A-29B & 30, 31-32 and 33A-33D are examples of apparatus with sensors 582 based on Figures 29B, 32B, 59 & 60A-60C, 69 & 70, 61A-61B & 62, 78 and 79 from the applicant's '916 U.S application and Figure 23A-C & G of US 2009/0200661 A1; Figures 25A and 26A are prior art examples based on Figures 29A and 32A of the '916 application.

### DETAILED DESCRIPTION OF EXAMPLE EMBODIMENTS

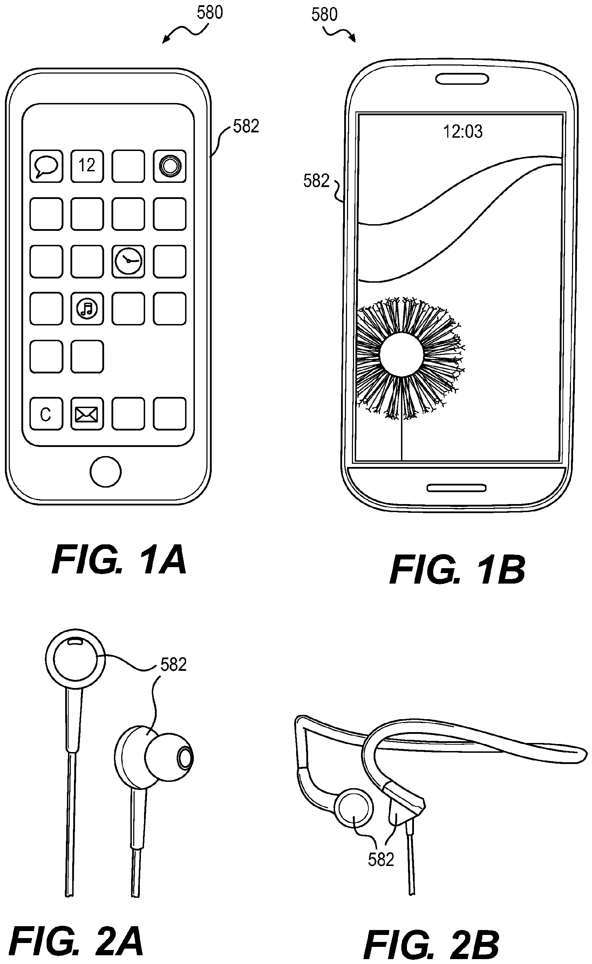

Among the inventions included in this application are the method, apparatus, and software of using a general or special purpose computer device 580, including a smartphone 580 such as an Apple iPhone 5™ and a Samsung Galaxy S III™, as shown in Figures 1A and 1B, as well as a Motorola Droid™, a Nokia Lumia™ and a BlackBerry™ 10, as other examples, to measure the relative motion of a body and/or a body part during the locomotion of a wearer of footwear or a user of the smartphone (or any other portable or mobile microcomputer device, general purpose or specialized, including for example the Apple iPod™).

The smartphone device 580 can also be used to measure the force or relative pressure distribution of the wearer's foot sole in footwear; or to do both, including simultaneously, while also performing the other standard functions of a smartphone, like performing phone, email, browsing, and audio and/or video functions.

Smartphones can be configured with existing hardware motion sensing components, like a three-axis gyroscope and three-axis accelerometer in the iPhone 5™ and Samsung Galaxy S III™, for example, and/or additional and/or new hardware or software components to perform similar or other motion data measurement. The above smartphone or other microcomputing devices 580 can be mobile and/or wearable.

The smartphone or other devices 580 can also be configured to be capable of recording and/or streaming (in real time or later) wired or wirelessly any such measurement data to another computer, either local to the user (using a tablet, laptop or desktop, for example) or to a cloud array of computers such as the example of the Apple iCloud or to any other computer. The smartphone or other device 580 can be configured to process the measurement data itself and/or in conjunction with a local and/or remote server, including the examples of an Apple iPhone™, a MacBook Pro™ laptop computer and/or the Apple iCloud™.

The smartphone or other device 580 can be configured to include the necessary sensor or sensors 582, in the device 580 itself and/or connected to it by wire or wirelessly, as well as associated electronic, software, and other components, to provide the capability to capture various kinds of motion and/or other data measurements; and/or the smartphone device can be used with one or more peripheral devices or apparatuses with sensors 582 and associated components, including one or more other mobile computer devices, general purpose or specialized, which can be located with or near the smartphone or located remotely from it, so that the motion of the body and/or one or more body parts of the wearer can be measured separately and/or simultaneously in whatever manner desired. For example, an iPhone 5™ with EarPods™ or other headphones, as shown in Figure 2A and 2B, that include motion sensors can be paired with an iPod™ with additional sensors and be connected to each other wirelessly or wired (even potentially in the future including the example of using a wearer's body as a conductive wire); and/or the iPhone5™ can receive video and/or audio input of the user during locomotion from another iPhone5™ while connected wirelessly.

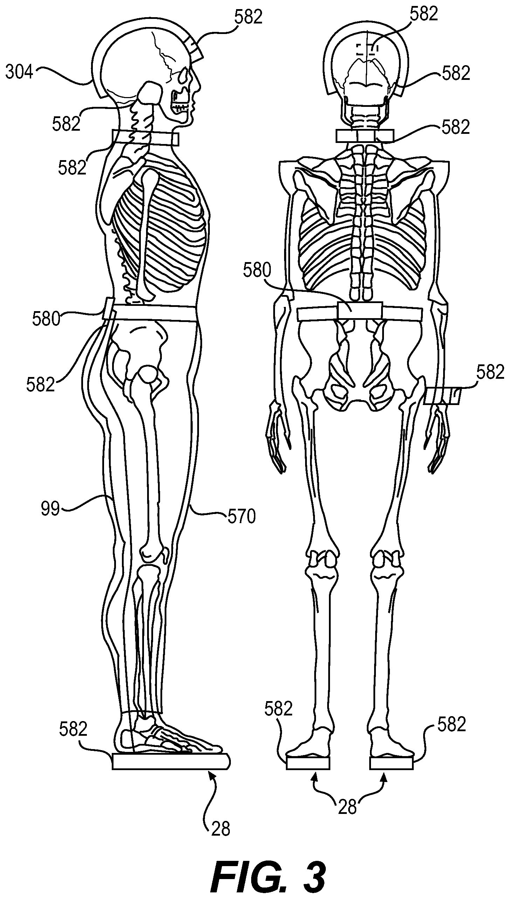

The smartphone 580 and/or associated peripheral devices or apparatuses with remote sensor or sensors 582 and/or other devices can be especially useful in measuring the relative motion of the body or one or more body parts of a user and/or wearer when the wearer is in any form of locomotion or gait, including walking and running. For example, an iPhone™ can be attached to a wearer's belt and located proximate to the small of the back of the wearer, as shown in Figure 3, at which location the iPhone™ is positioned close to the wearer's center of gravity (C.G.) and therefore data measurements of the wearer at that location during walking or running, for example, approximate fairly well the wearer's center of gravity (C.G.) motion during that locomotion, such as lateral or side-to-side C.G. motion in a frontal plane.

Besides the smartphone 580 being carried by the user/wearer, it can be maintained in a relative position close enough to the user/wearer that the smartphone can communicate wirelessly with one or more peripheral devices with sensors 582 on a test subject not carrying the smartphone during a test; alternatively, another computer like a tablet or laptop or desktop can be configured to perform the same functions as a smartphone device 580, although with less or little mobility; such a peripheral device with sensors 582 can itself also simply record data measurements for later transfer to the smartphone 580 or other computer in a wired or wireless connection or in a memory card like a USB drive or SD card, for examples.

In addition, the smartphone device 580 can be configured so that when it is paired with another motion sensor besides that in the smartphone or other device 580 itself, such as a separate sensor or sensors 582 in a peripheral device like an EarPod™ set or headphones; for example, that second sensor or sensors 582 can be configured to measure 1D, 2D, or 3D data of the relative motion of the wearer's head during locomotion (assuming earplug-like fixation by the ear canal) and that data can be recorded by and/or streamed to the smartphone device 580. The smartphone 580 such as an Apple iPhone™ can be configured to receive the data set of the head motion and compare that head motion data set with its own CG motion data set and/or send that head motion data set and/or its CG motion data set to the Apple iCloud for such comparison and/or both iPhone™ and iCloud™ can share the data set comparison and perform other functions in a shared operation and/or with either performing any given operation or part thereof independently.

The results of that motion data comparison and any other operation can be recorded and/or stored and/or transmitted or otherwise made accessible to the user/wearer and/or to an approved third party such as a doctor or podiatrist or biomechanics specialist or other professional or semi-professional technician, including licensed or not, who is treating, consulting, or evaluating the user/wearer for overuse injury or acute injury (including accidents of any type) or to prevent injury and/or optimize performance for locomotion including for work, sports, leisure, or activities of daily living. There can be any practical number of additional and/or separate motion or other sensors 582 of any useful configuration that can be located at any practical number of body parts of a user/wearer. The sensors 582 can be attached, fastened, or worn in any practical manner, including implanting in a human body (or in an animal body or in a plant). The smartphone or other device 580 can also be configured to receive shared data from other smartphones or other devices 580 and/or other shared peripheral devices with sensors 582 located with other user/wearers.

The smartphone or other device 580 can also measure absolute or geographic position of the wearer, such as by the global positioning system (GPS), compass, or other system, method or component, and can record and/or correlate in real time the absolute position (CG or other approximate point) of the user/wearer with the relative position motion of one or many body parts of the user/wearer (or the same information shared from other smartphone 580 user/wearers), and/or stream in real time or transmit the data sets to a cloud like the iCloud™ example above.

The position measurement, either relative or absolute, can be in one plane (1D) or in two planes (2D) or in three planes (3D), with correlated time measurement for example as well; sagittal, horizontal, and/or transverse (or frontal) planes are examples. Other data sets can potentially be captured, recorded, processed and/or transmitted by the smartphone or other device 580 or connected sensor or sensors 582, such as blood pressure, heart rate, respiration rate, blood suger level, weight, body temperature (core or a body part), ambient temperature, or any other body or body part measurement, medical or other.

The sensor or sensors 582 can be of any type, including the examples of relative and absolute motion, pressure, force, time, heat, moisture, chemical, electrical or electromagnetic, including visible light. The sensors 582 can be located in any practical location on any article of apparel or personal equipment, including the examples of earphones or earplugs, headphones, hat, helmet, protective padding or armor, braces, prosthetics, glasses including eyeglasses and Google Glass™, watch, belt, waistband, armband, attached with tape or bandage or glue, necklace or lanyard, cervical collar, ring, headband, in any manner attached or embedded in conventional or specialized clothing. The sensors 582 can also be worn or attached onto or implanted in the wearer's body, temporarily in a body piercing or permanently in a body implant.

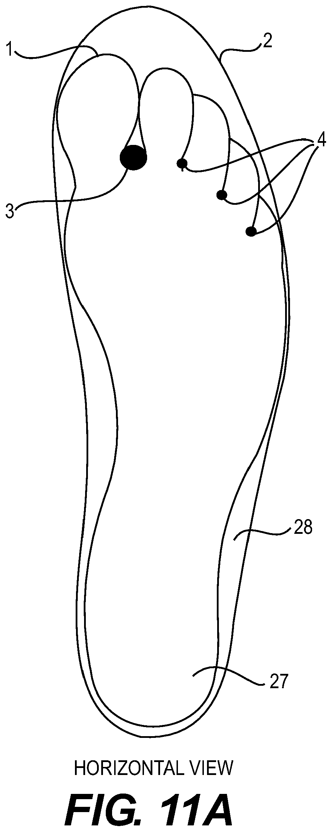

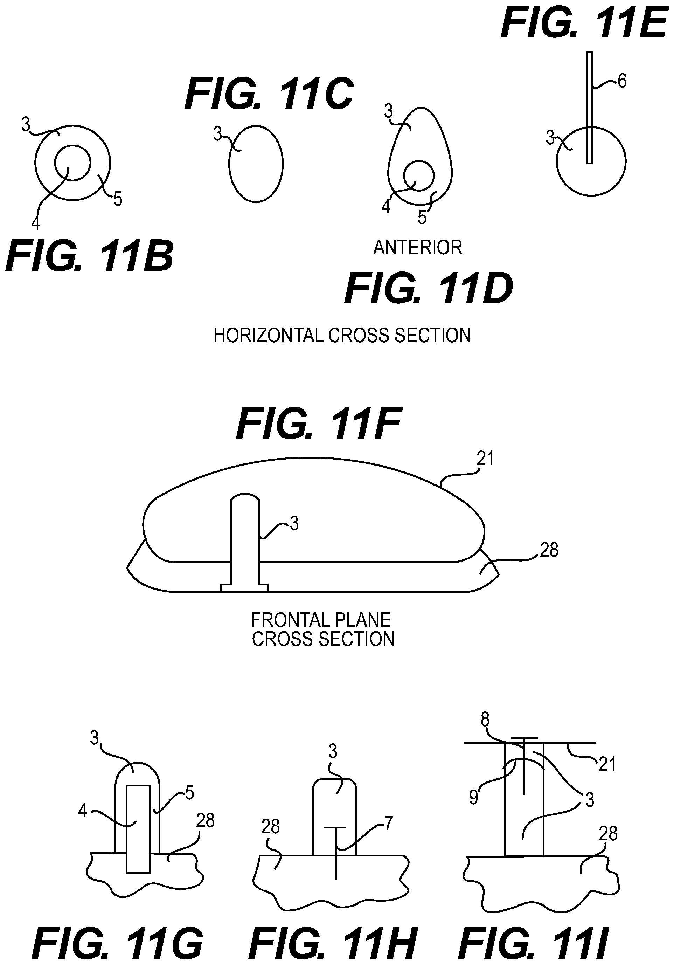

The sensors 582 can also be located in the wearer's footwear of any form or type, including in orthotics or prosthetics. The sensors 582 can be entirely diagnostic, such as the example of dynamic footsole force and/or pressure sensors 104 like F-Scan™ and similar in-shoe products like insoles. The footwear sensors 582 can also work with active foot motion control devices, like the example of computer controlled compartments located in or on footwear soles and/or removable in-shoe inserts like the examples shown in Figures 11A-11C, 11M, 11N, 11O, and IIP in the '350 Patent and Figures 11A-11C, 11M, 11N, 11O, 11P, 11T, 11U, 97, 98, and 99A & B in the '665 patent application; the U.S. Patent 5,813,142 to Demon is another example of the prior art. The example embodiments shown in these figures can be used to proactively and/or reactively alter shoe or orthotic (or prosthetic) soles to control the relative foot position between the right and left feet of the user/wearer's feet, such as to alter the neutral position of either or both feet separately toward a more generally supinated or pronated position, or for another example to alter the relative height of any specific portion of the right and/or left shoe or orthotic or prothetic sole, such as the forefoot, heel, or midsection or under any one or more bones of a user/wearer's right and/or left feet, or under the full right or left foot. These sole configuration alterations can be set potentially by smartphone device control for any time period, including dynamically for each step during locomotion of each foot of the user/wearer and/or dynamically many times during each step, and a data record of the alterations can be recorded and/or streamed from within each sole or in the sensor 582 or in the smartphone 580.

These footwear or orthotic sole alterations can be controlled by the smartphone device 580 based its 1D or 2D or 3D motion measurements, for example, of the user/wearer's body part or parts, including center of gravity motion (CG) during some form of locomotion. One example would be to correct for excessive lateral movement of the user/wearer's center of gravity to one side more than another, as measured in the frontal plane, compared to an established norm that is less prone to injury. Another example, which can be related, is to reduce the crossover of right and/or left extremities (legs and/or feet) across the centerline of the user/wearer's body, as measured in the wearer's frontal plane during locomotion. Pre-programmed solutions can be applied using the user/wearer's smartphone and/or a cloud, and real time or subsequent testing can be conducted, including by the third parties like a doctor or other professional or technician referenced earlier, by using the smartphone, including to connect directly to the third party or parties or to a cloud for shared or independent operations.

The operating systems of the above described smartphone or other device 580 can be an iOS™ or Android™ or Windows Phone OS or BlackBerry 10 for an Apple™ or Android™ or Windows™ or Linux™ smartphone or tablet, for example; other operating systems, existing or future can be used to perform those operations. Such an app can be downloaded from Apple™ or Google™, for example, or for a Kindle™ downloaded from Amazon™ or downloaded from Microsoft™ for the Nokia Lumia™ for other examples. The operations described above for one or more sensors 582 can also be controlled by the same app as for the smartphones or other device 580 above. The app can be software alone or include one or more special or new sensors 582 and/or other hardware and/or firmware.

At least the footwear specific portions of the app can be developed by a footwear vendor like Nike™, Adidas™, or Under Armor™, for example, and/or an independent or university biomechanics laboratory, and the overall app can be co-developed with the smartphone 580 and sensor 582 hardware makers, as well as the smartphone operation system developers, of which examples have been cited above. Data sets from the smartphone or other devices 580 can be transmitted to a World Wide Web site for processing, evaluation (especially comparison with other wearers or users), storage, sharing, and other functions for the user/wearer of the smartphone 580 (and may include the use of cloud resources) run by any of the entities referenced above, for example.

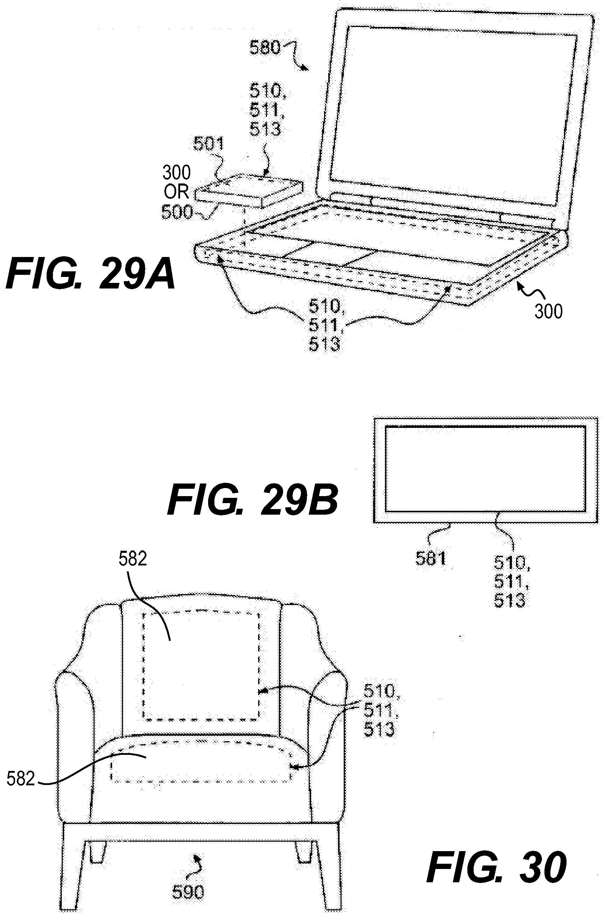

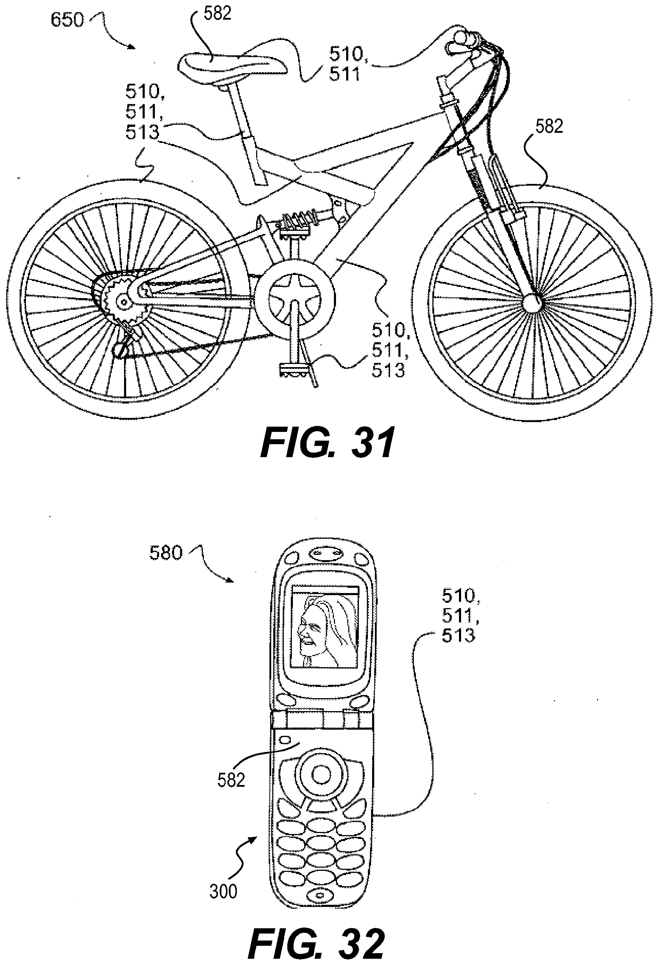

The smartphone or other device 580 can also be configured to control compartments implanted within (or attached to) the human body (or an animal body or plant), including examples such as Fig. 29A & B or Fig. 32A & B with multiple compartments like those previously shown for footwear, such as in Figs. 11M-P, 11T-U, and 97-99A & B of the '665 patent application. Such implants or attachments can be configured to include one or more sensors 582 discussed previously. Similarly, the smartphone or other device 580 can be used to control compartments in the same way in body braces, padding, and armor, including in the examples shown in Figures 60A, 69, and 70 of the '930 patent application.

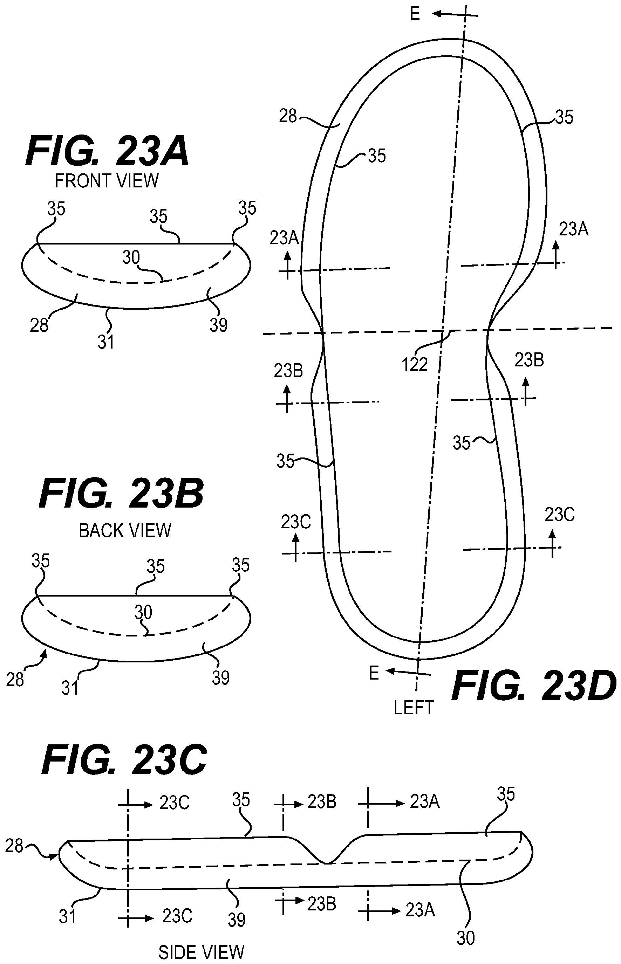

The computer or other device 580 including the example of a smartphone 580 can also be configured to include a microprocessor alone, including a system-on-a-chip (SoC), including a personal computer on a chip, and can also include a Faraday Cage 300, which can coincide with an outer compartment 500, such as the example shown in Fig. 23G of the '769 patent application. Especially because of privacy of data concerns, the smartphone or other device 580 or any Web site storing data therefrom can be configured to include any combination of security features indicated in the applicant's U.S. Patent Application No. 398,403 filed February 16, 2012,and published as Publication No. 20120311690 on December 6, 2012 and in Application No. 10,684,657 filed October 15, 2003 and published as Publication No. 2005/0180095 on August 18, 2005, both of which applications are hereby incorporated by reference in their entirety in this application.

A smartphone or other mobile computer device, either general purpose or specialized, can comprise the following: the smartphone device can be configured to actively control the configuration of one or more bladders, compartments, chambers or internal sipes and one or more sensors located in either one or both of a sole or a removable sole insert or inner sole insert of the footwear of the wearer and/or located in an apparatus worn or carried by the wearer, attached to the wearer, or implanted in the wearer; and the one or more bladders, compartments, chambers, or sipes, and one or more sensors can be configured for computer control. Any of the components or methods of the example invention embodiments described in this paragraph can be combined with any other components or methods of the example invention embodiments described in previous paragraphs above or in the patents or applications incorporated by reference in this application.

The smartphone device can be configured to record a first test data set consisting of measurements by a sensor of the force and/or the relative pressure distribution of a wearer's foot sole on or near an upper surface of the wearer's footwear during the wearer's locomotion or other physical activity; the first test data set as measured in at least one plane (1D) or in two planes (2D) or in three planes (3D) and/or including time or other measurements. Any of the components or methods of the example invention embodiments described in this paragraph can be combined with any other components or methods of the example invention embodiments described in previous paragraphs above or in the patents or applications incorporated by reference in this application.

The smartphone device can be configured to record a first test data set consisting of measurements by a sensor of the relative motion during the wearer's locomotion or other physical activity of a position at or near to a part of the body of the wearer; the first test data set as measured in at least one plane (1D) or in two planes (2D) or in three planes (3D) and/or including time or other measurements. Any of the components or methods of the example invention embodiments described in this paragraph can be combined with any other components or methods of the example invention embodiments described in previous paragraphs above or in the patents or applications incorporated by reference in this application.

The smartphone device can be configured to record a first test data set consisting of measurements of the relative motion during the wearer's locomotion or other physical activity of a position that is at or near the center of gravity of the body of the wearer, as measured in at least one plane (1D) or in two planes (2D) or in three planes (3D) and/or including time or other measurements. Any of the components or methods of the example invention embodiments described in this paragraph can be combined with any other components or methods of the example invention embodiments described in previous paragraphs above or in the patents or applications incorporated by reference in this application.

The smartphone device can be configured to establish a first configuration setting for the bladders, compartments, chambers, sipes or other portions of the apparatus or of either or both of the footwear soles. Any of the components or methods of the example invention embodiments described in this paragraph can be combined with any other components or methods of the example invention embodiments described in previous paragraphs above or in the patents or applications incorporated by reference in this application.

The first configuration setting of the smartphone device can be a neutral or baseline condition, including the condition wherein the smartphone device has not activated control of the apparatus or the footwear soles. Any of the components or methods of the example invention embodiments described in this paragraph can be combined with any other components or methods of the example invention embodiments described in previous paragraphs above or in the patents or applications incorporated by reference in this application.

The smartphone device can be configured to activate a second configuration setting for the bladders, compartments, chambers, sipes, or other portions of the apparatus or of either or both of the soles, the second configuration being different from the first configuration setting. Any of the components or methods of the example invention embodiments described in this paragraph can be combined with any other components or methods of the example invention embodiments described in previous paragraphs above or in the patents or applications incorporated by reference in this application.

Using the second configuration setting, the smartphone device can be configured to record a second test data set consisting of measurements of the relative motion during locomotion or other physical activity of the position at or near to the part of the wearer, as measured in at least one plane (1D) or in two planes (2D) or in three planes (3D) and/or including time or other measurements. Any of the components or methods of the example invention embodiments described in this paragraph can be combined with any other components or methods of the example invention embodiments described in previous paragraphs above or in the patents or applications incorporated by reference in this application.

Using the second configuration setting, the smartphone device can be configured to record a second test data set consisting of measurements of the relative motion during locomotion or other physical activity of the position at or near to the center of gravity of the wearer, as measured in at least one plane (1D) or in two planes (2D) or in three planes (3D) and/or including time or other measurements. Any of the components or methods of the example invention embodiments described in this paragraph can be combined with any other components or methods of the example invention embodiments described in previous paragraphs above or in the patents or applications incorporated by reference in this application.

The smartphone device can be configured to compare the first test data set and the second test data set with a preferred data set for the measurements of relative motion during locomotion or other physical activity of the part of a model wearer or wearers, as measured in at least one plane (1D) or in two planes (2D) or in three planes (3D) and/or including time or other measurements. Any of the components or methods of the example invention embodiments described in this paragraph can be combined with any other components or methods of the example invention embodiments described in previous paragraphs above or in the patents or applications incorporated by reference in this application.

The smartphone device can be configured to compare the first test data set and the second test data set with a preferred data set for the measurements of relative motion during locomotion or other physical activity of the position at or near to the part of a model wearer or wearers, as measured in at least one plane (1D) or in two planes (2D) or in three planes (3D) and/or including time or other measurements. Any of the components or methods of the example invention embodiments described in this paragraph can be combined with any other components or methods of the example invention embodiments described in previous paragraphs above or in the patents or applications incorporated by reference in this application.

The smartphone device can be configured to compare the first test data set and the second test data set with a preferred data set for the measurements of relative motion during locomotion or other physical activity of the position at or near to the center of gravity of a model wearer or wearers, as measured in at least one plane (1D) or in two planes (2D) or in three planes (3D) and/or including time or other measurements. Any of the components or methods of the example invention embodiments described in this paragraph can be combined with any other components or methods of the example invention embodiments described in previous paragraphs above or in the patents or applications incorporated by reference in this application.

The smartphone device can be configured to select the configuration setting of the footwear soles that produced the test data set that is the closest to the preferred data set and to reject the other configuration setting, thereby completing at least one full cycle of an operation to optimize the configuration for the wearer. Any of the components or methods of the example invention embodiments described in this paragraph can be combined with any other components or methods of the example invention embodiments described in previous paragraphs above or in the patents or applications incorporated by reference in this application.

The full cycle of the configuration optimizing operation can be repeated as frequently as necessary until the most recent test data set either closely matches the preferred data set or cannot be made to match the test data more closely. Any of the components or methods of the example invention embodiments described in this paragraph can be combined with any other components or methods of the example invention embodiments described in previous paragraphs above or in the patents or applications incorporated by reference in this application.

The full cycle of the configuration optimizing operation can be repeated hundreds or thousands or millions or billions of times. Any of the components or methods of the example invention embodiments described in this paragraph can be combined with any other components or methods of the example invention embodiments described in previous paragraphs above or in the patents or applications incorporated by reference in this application.

The model wearer or wearers can be chosen from a group of shod or barefoot wearers who have a history of low levels of overuse and/or acute injuries, the barefoot wearers including wearers that are distinguished by level of previous or current conventional footwear use, such as barefoot wearers that have been formerly shod and/or occasionally shod or seldom shod or never shod with conventional footwear. Any of the components or methods of the example invention embodiments described in this paragraph can be combined with any other components or methods of the example invention embodiments described in previous paragraphs above or in the patents or applications incorporated by reference in this application.

The smartphone device can be configured to include a gyroscope and an accelerometer. Any of the components or methods of the example invention embodiments described in this paragraph can be combined with any other components or methods of the example invention embodiments described in previous paragraphs above or in the patents or applications incorporated by reference in this application.

The smartphone device can be configured to include one or more wired connections and/or one or more wireless connections, the wireless connections including WiFi, Bluetooth, near field communications (NFC) and/or cellular. Any of the components or methods of the example invention embodiments described in this paragraph can be combined with any other components or methods of the example invention embodiments described in previous paragraphs above or in the patents or applications incorporated by reference in this application.

The relative motion can include geographic motion tracking between one or more geographic positions and said device is configured to include a global positioning system (GPS) components and/or another geographic location tracking capability. Any of the components or methods of the example invention embodiments described in this paragraph can be combined with any other components or methods of the example invention embodiments described in previous paragraphs above or in the patents or applications incorporated by reference in this application.

The smartphone device can be configured for wired and/or wireless connection to at least one peripheral device with at least one remote sensor located at or near to a body part of the wearer. Any of the components or methods of the example invention embodiments described in this paragraph can be combined with any other components or methods of the example invention embodiments described in previous paragraphs above or in the patents or applications incorporated by reference in this application.

The remote sensor can be of any known type, including motion, pressure, time, heat moisture, chemical, electrical, or electromagnetic sensor. At least one peripheral device can be a headphone set or a audio earplugs set or an earplugs set with at least one or two remote motion sensors. Any of the components or methods of the example invention embodiments described in this paragraph can be combined with any other components or methods of the example invention embodiments described in previous paragraphs above or in the patents or applications incorporated by reference in this application.

At least one peripheral device with at least one remote sensor can be configured to record and/or transmit a first and/or second test data set consisting of the measurements of the relative motion during locomotion or other physical activity of a position at or near to the body part of the wearer, as measured in at least one plane (1D) or in two planes (2D) or in three planes (3D) and/or including time or other measurements. Any of the components or methods of the example invention embodiments described in this paragraph can be combined with any other components or methods of the example invention embodiments described in previous paragraphs above or in the patents or applications incorporated by reference in this application.

The body part can be one or more of the wearer's head, neck, shoulder, chest, cervical, thoracic or lumbar back, rib, elbow, wrist, hand, waist, sacrum, pubic bone, illiac crest, thigh, hip, knee, patella, shin bone or tibia, ankle, toe, forefoot, midfoot or heel of foot; or wherein the body part is part of the body of an animal or a portion of a plant. Any of the components or methods of the example invention embodiments described in this paragraph can be combined with any other components or methods of the example invention embodiments described in previous paragraphs above or in the patents or applications incorporated by reference in this application.

The remote sensor can be located in any practical location on any article of clothing or personal equipment, including earphones or earplugs, helmet, glasses including eyeglasses and Google Glass™, watch, belt, waistband, elastic underwear, armband, attached with tape or bandage, necklace or lanyard, cervical collar, ring, headband, in any manner attached or embedded in conventional or specialized clothing, or attached to the skin of the wearer. Any of the components or methods of the example invention embodiments described in this paragraph can be combined with any other components or methods of the example invention embodiments described in previous paragraphs above or in the patents or applications incorporated by reference in this application.

The at least one peripheral device with at least one remote sensor can transmit, in real time and/or later, the first and/or second data sets to the smartphone device and/or to another computer. Any of the components or methods of the example invention embodiments described in this paragraph can be combined with any other components or methods of the example invention embodiments described in previous paragraphs above or in the patents or applications incorporated by reference in this application.

The apparatus or either or both of the footwear soles can include one or more or a multitude or at least 20 or 50 or 100 or 500 or 1000 or 4000 or 16,000 individual sensors. Any of the components or methods of the example invention embodiments described in this paragraph can be combined with any other components or methods of the example invention embodiments described in previous paragraphs above or in the patents or applications incorporated by reference in this application.

The locomotion can include walking and/or running. The test data sets can include at least a full stride or many strides of the walking and/or running locomotion or at least one full cycle or many cycles of any other repetitive motion of the wearer. The first and/or second test data sets can be collected when the locomotion occurs on a flat level surface, a flat uphill or upward inclining surface, or a flat downhill or downward inclining surface. Any of the components or methods of the example invention embodiments described in this paragraph can be combined with any other components or methods of the example invention embodiments described in previous paragraphs above or in the patents or applications incorporated by reference in this application.

The apparatus or either or both of the footwear soles can be configured to include at least a magnetorheological fluid located in the one or more bladders, compartments, chambers, sipes or other portions, the magnetorheological fluid being controlled at least in part or completely by the smartphone device. Any of the components or methods of the example invention embodiments described in this paragraph can be combined with any other components or methods of the example invention embodiments described in previous paragraphs above or in the patents or applications incorporated by reference in this application.

The apparatus or either or both of the footwear soles can be configured to include at least one valve located between the two or more bladders, compartments, chambers, sipes, or other portions, the at least one valve being controlled at least in part or completely by the smartphone device. Any of the components or methods of the example invention embodiments described in this paragraph can be combined with any other components or methods of the example invention embodiments described in previous paragraphs above or in the patents or applications incorporated by reference in this application.

The apparatus or either or both of the footwear soles can be configured to include at least one electric and/or electronic and/or electromechanical device that is controlled at least in part or completely by the smartphone device Any of the components or methods of the example invention embodiments described in this paragraph can be combined with any other components or methods of the example invention embodiments described in previous paragraphs above or in the patents or applications incorporated by reference in this application..

The apparatus and/or footwear sole or soles can include at least one battery and/or at least one device wherein the body weight and/or muscular energy of a wearer of the smartphone device is used to generate electrical power in the apparatus or either or both of the footwear soles. Any of the components or methods of the example invention embodiments described in this paragraph can be combined with any other components or methods of the example invention embodiments described in previous paragraphs above or in the patents or applications incorporated by reference in this application.

The apparatus or either or both of the footwear soles can be configured to include a wired and/or wireless connection to the smartphone device. Any of the components or methods of the example invention embodiments described in this paragraph can be combined with any other components or methods of the example invention embodiments described in previous paragraphs above or in the patents or applications incorporated by reference in this application.

An article of apparel or equipment can be configured to include wiring to connect the smartphone device to the apparatus and/or either or both of the footwear soles and/or one or more peripheral devices with at least one remote senor; and/or wherein the smartphone device is configured to provide power to the apparatus and/or footwear soles and/or peripheral devices. Any of the components or methods of the example invention embodiments described in this paragraph can be combined with any other components or methods of the example invention embodiments described in previous paragraphs above or in the patents or applications incorporated by reference in this application.

The smartphone device can be configured to actively control the configuration of one or more footwear soles of the wearer by altering the relative longitudinal height, including positive or negative heel lift (or drop), or negative or positive forefoot lift, and/or the relative side-to-side height between lateral and medial sides, and/or the relative height between the right and the left footwear, or a combination of these relative height alterations. These alterations are structurally and functionally like those performed typically by podiatrists and orthopedic specialists, for example. Any of the components or methods of the example invention embodiments described in this paragraph can be combined with any other components or methods of the example invention embodiments described in previous paragraphs above or in the patents or applications incorporated by reference in this application.

The smartphone device can be configured to actively control the configuration of one or more footwear soles of the wearer by altering the relative longitudinal firmness between heel area and forefoot area and/or side-to-side firmness between lateral and medial side areas, and/or the relative firmness between the right and the left footwear, or a combination of these relative firmness alterations. These alterations are structurally and functionally like those performed typically by podiatrists and orthopedic specialists, for example. Any of the components or methods of the example invention embodiments described in this paragraph can be combined with any other components or methods of the example invention embodiments described in previous paragraphs above or in the patents or applications incorporated by reference in this application.

The smartphone device can be configured to actively control the configuration of one or more footwear soles of the wearer by altering the relative height or firmness under one or more of the foot bones of the wearer, including under the calcaneus, the lateral calcaneal tuberosity, the base of the fifth metatarsal, the longitudinal arch, the metatarsal arch, each of the heads of the metatarsals, and each of the distal phalanges, including the hallux or big toe. These alterations are structurally and functionally like those performed typically by podiatrists and orthopedic specialists, for example. Any of the components or methods of the example invention embodiments described in this paragraph can be combined with any other components or methods of the example invention embodiments described in previous paragraphs above or in the patents or applications incorporated by reference in this application.

The smartphone device can be configured to actively control of the apparatus or footwear configuration at least once per full operation cycle or locomotion stride, many times per full operation cycle or locomotion stride, once per many full operation cycles or locomotion strides, or based on a set time period of any duration or based on another test condition. Any of the components or methods of the example invention embodiments described in this paragraph can be combined with any other components or methods of the example invention embodiments described in previous paragraphs above or in the patents or applications incorporated by reference in this application.

The device can be configured to record a first test data set consisting of measurements of the force and/or the relative pressure distribution of the wearer's foot sole on an upper surface of the footwear during the wearer's locomotion or other physical activity, as measured in at least one plane (1D) or in two planes (2D) or in three planes (3D) and/or including time or other measurements, the footwear upper surface including at least a multitude or at least 20 or 50 or 100 or 500 or 1,000 or 4,000, or 16,000 individual pressure sensors. Any of the components or methods of the example invention embodiments described in this paragraph can be combined with any other components or methods of the example invention embodiments described in previous paragraphs above or in the patents or applications incorporated by reference in this application.

Using the second configuration setting, the smartphone device can be configured to record a second test data set consisting of measurements of the force and/or the relative pressure distribution of the wearer's footsole on an upper surface of the footwear during the wearer's locomotion or other physical activity, as measured in at least one plane (1D) or in two planes (2D) or in three planes (3D) and/or including time or other measurements. Any of the components or methods of the example invention embodiments described in this paragraph can be combined with any other components or methods of the example invention embodiments described in previous paragraphs above or in the patents or applications incorporated by reference in this application.

The smartphone device can be configured to compare the first test data set and the second test data set with a preferred data set for the measurements of force and/or relative pressure distribution of the foot sole of a model wearer or wearers on an upper surface of the footwear during the locomotion or other physical activity, as measured in at least one plane (1D) or in two planes (2D) or in three planes (3D) and/or including time or other measurements. Any of the components or methods of the example invention embodiments described in this paragraph can be combined with any other components or methods of the example invention embodiments described in previous paragraphs above or in the patents or applications incorporated by reference in this application.

The smartphone device can be configured to select the configuration setting of the soles that produced the test data set for the force or relative pressure distribution that is the closest to the preferred data set for force or relative pressure distribution and to reject the other configuration setting, thereby completing at least one full cycle of an operation to optimize the wearer's configuration. Any of the components or methods of the example invention embodiments described in this paragraph can be combined with any other components or methods of the example invention embodiments described in previous paragraphs above or in the patents or applications incorporated by reference in this application.

The optimizing operation can be used to reduce a range of pronation and/or supination of the wearer's foot and ankle during the landing phase of locomotion through active configuration by the smartphone device of the either or both of the footwear soles. Any of the components or methods of the example invention embodiments described in this paragraph can be combined with any other components or methods of the example invention embodiments described in previous paragraphs above or in the patents or applications incorporated by reference in this application.

The optimizing operation can be used by the smartphone device to actively configure either or both of the footwear soles or the apparatus in one or more or many areas of high and/or low pressure as measured on the upper surface of the footwear soles during the landing phase of locomotion or as measured on the outer surface of the apparatus during operation. Any of the components or methods of the example invention embodiments described in this paragraph can be combined with any other components or methods of the example invention embodiments described in previous paragraphs above or in the patents or applications incorporated by reference in this application.

The optimizing operation can be used by the smartphone device to actively configure either or both of the footwear soles or the apparatus to produce a forefoot strike, a midfoot strike, or a heel strike at the beginning of the landing phase during locomotion for either or both of the wearer's feet. Any of the components or methods of the example invention embodiments described in this paragraph can be combined with any other components or methods of the example invention embodiments described in previous paragraphs above or in the patents or applications incorporated by reference in this application.

The optimizing operation can be used by the smartphone device to actively configure either or both of the footwear soles or the apparatus to change the motion of the center of force on the surface of footwear for either or both of the wearer's feet during locomotion. Any of the components or methods of the example invention embodiments described in this paragraph can be combined with any other components or methods of the example invention embodiments described in previous paragraphs above or in the patents or applications incorporated by reference in this application.

Other test data sets can potentially be monitored, recorded, processed and/or transmitted by the smartphone device or remote sensor or sensors, such as blood pressure, heart rate, respiration rate, blood sugar level, weight, body temperature (core or a body part), ambient temperature, or any other body or body part measurement, medical or other, or audio or video. Any of the components or methods of the example invention embodiments described in this paragraph can be combined with any other components or methods of the example invention embodiments described in previous paragraphs above or in the patents or applications incorporated by reference in this application.

One or more of the test data sets can be transmitted to a cloud system for storage and/or shared or independent processing and/or analysis of groups or categories of users and/or shared access by permitted third parties and by the user. Any of the components or methods of the example invention embodiments described in this paragraph can be combined with any other components or methods of the example invention embodiments described in previous paragraphs above or in the patents or applications incorporated by reference in this application.

One or more of the test data sets can be transmitted to a web site for storage and/or processing and/or analysis of groups or categories of users and/or shared access by the user and by third parties permitted by the user. Any of the components or methods of the example invention embodiments described in this paragraph can be combined with any other components or methods of the example invention embodiments described in previous paragraphs above or in the patents or applications incorporated by reference in this application.

The smartphone device can be used to measure the relative positions to each other of a wearer's right and left feet during the stance phase of locomotion so as to determine the degree of crossover of right and/or left feet across the centerline of the wearer's body, as measured in the frontal plane during the stance phase of locomotion; and then to test a series of configuration settings in order to reduce or eliminate the crossover. Any of the components or methods of the example invention embodiments described in this paragraph can be combined with any other components or methods of the example invention embodiments described in previous paragraphs above or in the patents or applications incorporated by reference in this application.

The smartphone device and/or the apparatus and/or the footwear and/or the peripheral devices with sensors can be used as a medical system or a medical tool for diagnostic, therapeutic, and/or rehabilitative functions before and/or during and/or after surgical or other medical treatment. Any of the components or methods of the example invention embodiments described in this paragraph can be combined with any other components or methods of the example invention embodiments described in previous paragraphs above or in the patents or applications incorporated by reference in this application.

The smartphone device and/or the apparatus and/or the footwear and/or the peripheral devices with sensors can be used as a medical system or a medical tool for medical treatment functions through non-surgical means. Any of the components or methods of the example invention embodiments described in this paragraph can be combined with any other components or methods of the example invention embodiments described in previous paragraphs above or in the patents or applications incorporated by reference in this application.

The smartphone device and/or the apparatus and/or the footwear and/or the peripheral devices with sensors can be used as a podiatric system or a podiatric tool for diagnostic, therapeutic, and/or rehabilitative functions before and/or during and/or after surgical or other podiatric treatment. Any of the components or methods of the example invention embodiments described in this paragraph can be combined with any other components or methods of the example invention embodiments described in previous paragraphs above or in the patents or applications incorporated by reference in this application.

The smartphone device and/or the apparatus and/or the footwear and/or the peripheral devices with sensors can be used as a medical system or a medical tool to stimulate or retard structural bone growth and/or joint development in a child wearer prior to adulthood through non-surgical means. Any of the components or methods of the example invention embodiments described in this paragraph can be combined with any other components or methods of the example invention embodiments described in previous paragraphs above or in the patents or applications incorporated by reference in this application.

The smartphone device and/or the apparatus and/or the footwear and/or the peripheral devices with sensors can be used as a medical system or a medical tool to prevent or reduce the gradual deterioration of bone and/or joint structure in an adult wearer through non-surgical means. Any of the components or methods of the example invention embodiments described in this paragraph can be combined with any other components or methods of the example invention embodiments described in previous paragraphs above or in the patents or applications incorporated by reference in this application.

The smartphone device and/or the apparatus and/or the footwear and/or the peripheral devices with sensors can be used as a medical system or a medical tool to treat the deterioration of bone and/or joint structure in an elderly wearer through non-surgical means. Any of the components or methods of the example invention embodiments described in this paragraph can be combined with any other components or methods of the example invention embodiments described in previous paragraphs above or in the patents or applications incorporated by reference in this application.

The smartphone device can be worn at the centerline of the rearmost portion of the wearer's belt or otherwise attached at or near the small of the wearer's lumbar back, centered between and at about the level of the illiac crests. Any of the components or methods of the example invention embodiments described in this paragraph can be combined with any other components or methods of the example invention embodiments described in previous paragraphs above or in the patents or applications incorporated by reference in this application.

The apparatus can be a helmet and/or helmet padding and/or other padding or protective gear, including braces, with one or more bladders, compartments, chambers, sipes, or other portions that are actively configured by the smartphone device. Any of the components or methods of the example invention embodiments described in this paragraph can be combined with any other components or methods of the example invention embodiments described in previous paragraphs above or in the patents or applications incorporated by reference in this application.

The footwear can be configured so that any part of or all of the configurable components of the footwear are located in a removable or fixed insert or a removable or fixed orthotic. Any of the components or methods of the example invention embodiments described in this paragraph can be combined with any other components or methods of the example invention embodiments described in previous paragraphs above or in the patents or applications incorporated by reference in this application.

One or both of a wearer's footwear can be configured to include a sole which has concavely rounded upper and lower surfaces relative to the intended wearer's foot sole, as measured in at least in a frontal plane cross-section taken in the heel area, the forefoot, and in the midfoot area; and/or wherein the upper and lower surfaces are substantially parallel; and/or wherein the concavely rounded lower surface extends to the lateral or sidemost extent 44 of one or both sides of the sole. Any of the components or methods of the example invention embodiments described in this paragraph can be combined with any other components or methods of the example invention embodiments described in previous paragraphs above or in the patents or applications incorporated by reference in this application.

One or both of a wearer's concavely rounded footwear can be configured to deform under the pressure of the wearer's body weight so as to flatten the rounding against the flat surface of the ground, in just the same way that rounded portions of the wearer's foot sole flatten against the flat surface of the ground. Any of the components or methods of the example invention embodiments described in this paragraph can be combined with any other components or methods of the example invention embodiments described in previous paragraphs above or in the patents or applications incorporated by reference in this application.

One or both of a wearer's footwear can be configured to include a sole which has a toe end portion and a heel end portion that have concavely rounded upper and lower surfaces relative to the position of the intended wearer's foot sole, as measured in at least in a sagittal plane cross-section taken along the long axis of the footwear; and/or wherein the upper and lower surfaces can be substantially parallel; and/or wherein the concavely rounded lower surface can extend to the most anterior extent and/or posterior extent of the sole; and/or one or both of a wearer's footwear can be configured to include a substantially flat portion between the toe end portion and heel end portion, as measured in at least in a sagittal plane cross-section taken along the long axis of the footwear. Any of the components or methods of the example invention embodiments described in this paragraph can be combined with any other components or methods of the example invention embodiments described in previous paragraphs above or in the patents or applications incorporated by reference in this application.

One or both sides of the sole can have at least one flexibility groove located in the midfoot of the footwear sole proximate to a flexibility axis 122 located at about the position posterior to the forefoot of the footwear sole and anterior to a position proximate to the base of the fifth metatarsal of the intended wearer's foot sole; and/or the flexibility grove can extend through part or all of the underneath portion between the sides of the footwear sides; and/or the footwear sole has other flexibility grooves. Any of the components or methods of the example invention embodiments described in this paragraph can be combined with any other components or methods of the example invention embodiments described in previous paragraphs above or in the patents or applications incorporated by reference in this application.

One or both of a wearer's concavely rounded footwear soles can be formed from a flat sheet of heat and/or pressure-sensitive plastic and/or rubber, including foamed or blown, the flat sheet being put under heat and/or pressure by a press 55 with upper and lower surfaces configured to produce the concavely rounded footwear sole; and/or the footwear sole can be configured to include at least two layers that are laminated together with heat and/or pressure sensitive glue; and/or at least a part or all of the footwear sole can be formed using a mold. Any of the components or methods of the example invention embodiments described in this paragraph can be combined with any other components or methods of the example invention embodiments described in previous paragraphs above or in the patents or applications incorporated by reference in this application.

One or both of a wearer's footwear sole can be configured to have at least a semi-thong 3 positioned to be located between the big toe and second toe of the intended wearer's foot; the semi-thong can be fixed, fastened, or embedded in only to the upper surface and/or other portions of the footwear sole and can be not fixed to and/or contacting a portion of the footwear upper or straps; and/or optional semi-thongs positioned can be to be located between one or more or all of the other toes of the wearer's foot sole; and/or the semi-thong can have a round, an oval, or an anthropomorphically-determined shape, as viewed in a horizontal cross-section; and/or the semi-thong can be constructed of plastic and/or rubber, including foamed or blown; and/or the semi-thong can be configured to have at least one softer material on the outer surface and a core of at least one firmer material inside; and/or the semi-thong can be configured to form a portion of the bottom surface the footwear sole; and/or the semi-thong can be configured to be temporarily fastened to at least a portion of the footwear upper or strap; and/or wherein the semi-thong can have a strut extending forward between the toes that serves as a protective partition between the toes. Any of the components or methods of the example invention embodiments described in this paragraph can be combined with any other components or methods of the example invention embodiments described in previous paragraphs above or in the patents or applications incorporated by reference in this application.

One or both of a wearer's most minimalist footwear can be a footwear sole and/or a sock that is configured to include only a fabric layer, a traction coating layer on the outer surface of the fabric, and a traction coating layer on the inner surface of the fabric; and/or wherein the coating layers can be rubber and/or plastic, including foamed and/or blown; and/or wherein one or both of the coating layers can be continuous or formed in a geometric or other pattern or randomly oriented and/or irregularly shaped; and/or the minimalist footwear can be configured to include a midsole insert and/or orthotic; and/or wherein one or both of a wearer's most minimalist footwear can be a footwear sole and/or a sock that is configured to include only the fabric, which is fabricated with a thread coated with a traction coating layer. Any of the components or methods of the example invention embodiments described in this paragraph can be combined with any other components or methods of the example invention embodiments described in previous paragraphs above or in the patents or applications incorporated by reference in this application.

One or both of a wearer's most minimalist footwear can be a footwear sole and/or a sock that can be configured to include at least one fabric layer, at least one traction coating layer on the outer surface of the fabric, and at least one traction coating layer on the inner surface of the fabric; and/or wherein the coating layers can be rubber and/or plastic, including foamed and/or blown; and/or wherein one or both of the coating layers can be continuous or formed in a geometric or other pattern or randomly can be oriented and/or irregularly shaped; and/or the minimalist footwear can be configured to include a midsole insert and/or orthotic; and/or wherein the fabric can be fabricated with a thread coated with a traction coating layer. Any of the components or methods of the example invention embodiments described in this paragraph can be combined with any other components or methods of the example invention embodiments described in previous paragraphs above or in the patents or applications incorporated by reference in this application.

The smartphone device or apparatus can be configured to include an outer coating of Teflon™. Any of the components or methods of the example invention embodiments described in this paragraph can be combined with any other components or methods of the example invention embodiments described in previous paragraphs above or in the patents or applications incorporated by reference in this application.

A software app as described above can configure at least a portion of a part or all of the configuration of a smartphone device and/or one or more sensors and/or one or both footwear and/or one or more apparatus. Any of the components or methods of the example invention embodiments described in this paragraph can be combined with any other components or methods of the example invention embodiments described in previous paragraphs above or in the patents or applications incorporated by reference in this application.

A sole and/or a removable sole insert or inner sole insert for footwear can comprise the following: one or more bladders, compartments, chambers, internal sipes or other portions located in the sole and/or in a removable insert; one or more sensors located in or on the sole and/or in the removable sole insert and/or located in or on an insole; the one or more bladders, compartments, chambers, sipes or other portions and the one or more sensors can be configured for control by a smartphone or other mobile computer device, general purpose or specialized; and/or the control can be conducted through a wired or a wireless connection. In addition, one or both of a wearer's footwear and/or the removable sole insert or inner sole insert for footwear can be configured to include a sole which has concavely rounded upper and lower surfaces relative to the intended wearer's foot sole, as measured in at least in a frontal plane cross-section taken in the heel area, the forefoot, and in the midfoot area; and/or the upper and lower surfaces can be substantially parallel; and/or the concavely rounded lower surface can extend to the lateral extent 44 of one or both sides of the sole; and/or the lateral extent 44 can extend above the lowest point of the inner footwear surface. Any of the components or methods of the example invention embodiments described in this paragraph can be combined with any other components or methods of the example invention embodiments described in previous paragraphs above or in the patents or applications incorporated by reference in this application.

A sole and/or a removable sole insert or inner sole insert for footwear for footwear can comprise the following: one or both of a wearer's footwear can be configured to include a sole and/or the removable sole insert or inner sole insert which has concavely rounded upper and lower surfaces relative to the intended wearer's foot sole, as measured in at least in a frontal plane cross-section taken in the heel area, in the forefoot area, and in the midfoot area; and/or the upper and lower surfaces can be substantially parallel; and/or the concavely rounded lower surface can extend to the lateral extent 44 of one or both sides of the sole; and/or the lateral extent 44 can extend above the lowest point of the inner footwear surface. Any of the components or methods of the example invention embodiments described in this paragraph can be combined with any other components or methods of the example invention embodiments described in previous paragraphs above or in the patents or applications incorporated by reference in this application.

A sole and/or a removable sole insert for footwear can comprise the following: one or both of a wearer's footwear sole and/or the removable sole insert can be configured to have at least a semi-thong 3 positioned to be located between the big toe and second toe of the intended wearer's foot; the semi-thong can be fixed, fastened, or embedded in only to the upper surface and/or other portions of the footwear sole and can be not fixed to and/or contacting a portion of the footwear upper or straps; and/or optional semi-thongs can be positioned to be located between one or more or all of the other toes of the wearer's foot sole; and/or the semi-thong can have a round, an oval, or an anthropomorphically-determined shape, as viewed in a horizontal cross-section; and/or the semi-thong can be constructed of plastic and/or rubber, including foamed or blown; and/or the semi-thong can be configured to have at least one softer material on the outer surface and a core of at least one firmer material inside; and/or the semi-thong can be configured to form a portion of the bottom surface the footwear sole; and/or the semi-thong can be configured to be temporarily fastened to at least a portion of the footwear upper or strap; and/or wherein the semi-thong can have a strut extending forward between the toes that serves as a protective partition between the toes. One or both of a wearer's footwear can be configured to include a sole which has concavely rounded upper and lower surfaces relative to the intended wearer's foot sole, as measured in at least in a frontal plane cross-section taken in the heel area, in the forefoot area, and in the midfoot area; and/or wherein the upper and lower surfaces can be substantially parallel; and/or wherein the concavely rounded lower surface can extend to the lateral extent 44 of one or both sides of the sole. Any of the components or methods of the example invention embodiments described in this paragraph can be combined with any other components or methods of the example invention embodiments described in previous paragraphs above or in the patents or applications incorporated by reference in this application.

A sole can comprise the following: one or both of a wearer's most minimalist footwear can be a footwear sole and/or a sock that can be configured to include only a fabric layer, a traction coating layer on the outer surface of the fabric, and a traction coating layer on the inner surface of the fabric; and/or wherein the coating layers can be a rubber and/or plastic, including foamed and/or blown; and/or wherein one or both of the coating layers can be continuous or formed in a geometric or other pattern or randomly oriented and/or irregularly shaped; and/or the minimalist footwear can be configured to include a midsole insert and/or orthotic; and/or wherein one or both of a wearer's most minimalist footwear can be a footwear sole and/or a sock that is configured to include only the fabric, which can be fabricated with a thread coated with a traction coating layer; and/or the sole is configure to allow for a removable sole insert as discussed at least in the several preceding three paragraphs. Any of the components or methods of the example invention embodiments described in this paragraph can be combined with any other components or methods of the example invention embodiments described in previous paragraphs above or in the patents or applications incorporated by reference in this application.

A sole can comprise the following: one or both of a wearer's most minimalist footwear can be a footwear sole and/or a sock that can be configured to include at least one fabric layer, at least one traction coating layer on the outer surface of the fabric, and at least one traction coating layer on the inner surface of the fabric; and/or wherein the coating layers can be rubber and/or plastic, including foamed and/or blown; and/or wherein one or both of the coating layers can be continuous or formed in a geometric or other pattern or randomly oriented and/or irregularly shaped; and/or the minimalist footwear can be configured to include a midsole insert and/or orthotic; and/or wherein the fabric can be fabricated with a thread coated with a traction coating layer; and/or the sole can be configured to allow for a removable sole insert as discussed above. Any of the components or methods of the example invention embodiments described in this paragraph can be combined with any other components or methods of the example invention embodiments described in previous paragraphs above or in the patents or applications incorporated by reference in this application.

An apparatus can comprise the following: one or more bladders, compartments, chambers, sipes or other portions can be located in the apparatus; one or more sensors can be located in or on the apparatus; the one or bladders, compartments, chambers, sipes or other portions and the one or more sensors being configured for control by a smartphone or other mobile computer device, general purpose or specialized; and/or the control can be conducted through a wired or a wireless connection. The apparatus can be configured to operate with and/or be controlled by the smartphone device as described in detail above. Any of the components or methods of the example invention embodiments described in this paragraph can be combined with any other components or methods of the example invention embodiments described in previous paragraphs above or in the patents or applications incorporated by reference in this application.

An article of apparel or equipment can comprise the following: the article of apparel or equipment can be configured to include wiring to connect the smartphone device to the apparatus and/or either or both of the footwear soles and/or one or more peripheral devices with at least one remote senor; and/or wherein the smartphone device can be configured to provide power to the apparatus and/or footwear soles and/or peripheral devices. Any of the components or methods of the example invention embodiments described in this paragraph can be combined with any other components or methods of the example invention embodiments described in previous paragraphs above or in the patents or applications incorporated by reference in this application.

A helmet, including faceguard and/or chinguard, or other protective equipment including braces and body armor can be configured to comprise an outer coating of Teflon™ 304 to reduce rotational forces such as on the head. Even a smartphone device or an apparatus can usefully be configured to comprise an outer coating of Teflon™ 304 to reduce forces when positioned inside a protective case; alternatively, a case or other protective device for a smartphone device or tablet or other electronic device can be configured to comprise an outer coating of Teflon™ to reduce tangential impact forces.

It must be noted that as used herein, the singular forms "a", "an", and "the" include plural references unless the context clearly indicates otherwise. Furthermore, the terms "a"(or "an"), "one or more" and "at least one" can be used interchangeably herein. The terms "comprising", "including", "having" and "constructed from" can also be used interchangeably.

Any of the footwear soles and/or removable sole inserts (either of which can be orthotics) described in the textual specification and/or the associated figures of this application, can have a single layer of one or more shoe sole compatible materials or multiple layers of one or more shoe sole compatible materials and can include an optional outsole or bottom sole layer, optionally, one or more midsole layers, optionally, one or more lifts in the forefoot, midfoot, or heel areas of the footwear sole or insert, and optionally, one or more insole or sock liner layers; and any layer can encompass one or more parts or all of the area of the shoe soles or removable sole inserts; and the shoe sole compatible materials can be a solid or can include a gas, such as foamed plastic or blown rubber.

The foregoing description has been presented for the purpose of illustration and description only and is not to be construed as limiting the invention in any way. The scope of the invention is to be determined from the claims appended hereto.

## Claims

1. A smartphone or other mobile computer device (580), general purpose or specialized, comprising:
a configuration of the smartphone or other mobile computer device (580) whereby the smartphone or other mobile computer device (580) can actively control the configuration of one or more bladders, compartments, chambers or internal sipes located in either one or both of a sole or a removable sole insert of the footwear of a wearer, the one or more bladders, compartments, chambers, or sipes being configured for computer control, **characterized in that**
the smartphone or other mobile computer device (580) actively controls the configuration of the one or more bladders, compartments, chambers or internal sipes based on input from one or more sensors (582) located in either one or both of a sole or a removable sole insert of the footwear of the wearer and from at least one sensor (582) selected from a gyroscope and an accelerometer located in the smartphone or other mobile computer device (580) or in another device or in an apparatus worn or carried by the wearer, attached to the wearer, or implanted in the wearer, the apparatus or the other device being separate from the footwear of the wearer.

2. The smartphone or other mobile computer device (580) of claim 1, wherein the smartphone or other mobile computer device (580) can actively control the configuration of one or more bladders, compartments, chambers or internal sipes based on input from one or more sensors (582) located in either one or both of a sole or a removable sole insert of the footwear of the wearer and a gyroscope and from an accelerometer independently located in the smartphone or other mobile computer device (580) or in the another device or in the apparatus.

3. The smartphone or other mobile computer device (580) of any one of claims 1-2, wherein the smartphone or other mobile computer device (580) can actively control in part or completely a magnetorheological fluid located in the one or more bladders, compartments, chambers or internal sipes; and/or the smartphone or other mobile computer device (580) can actively control in part or completely at least one valve located between two or more of said bladders, compartments, chambers or internal sipes; and/or the smartphone or other mobile computer device (580) can actively control in part or completely at least one electric and/or electronic and/or electromechanical device.

4. The smartphone or other mobile computer device (580) of any one of claims 1-3, wherein the smartphone or other mobile computer device (580) is configured to actively control the configuration of one or more footwear soles or removable sole inserts of the wearer by altering a relative longitudinal height, including positive or negative heel lift, or negative or positive forefoot lift, and/or a relative side-to-side height between lateral and medial sides, and/or a relative height between right and left footwear soles or removable sole inserts, or a combination of these relative height alterations; and/or
the smartphone or other mobile computer device (580) is configured to actively control the configuration of one or more footwear soles or removable sole inserts of the wearer by altering relative longitudinal firmness between heel area and forefoot area and/or side-to-side firmness between lateral and medial side areas, and/or relative firmness between a right and left footwear sole or removable sole insert, or a combination of these relative firmness alterations; and/or
the smartphone or other mobile computer device (580) is configured to actively control the configuration of one or more footwear soles or removable sole inserts of the wearer by altering relative height or firmness under one or more of the foot bones of the wearer, including under a calcaneus, a lateral calcaneal tuberosity, a base of a fifth metatarsal, each head of a metatarsal, and each distal phalange, including a hallux or big toe, or under a longitudinal arch or a metatarsal arch of the wearer; and/or
the smartphone or other mobile computer device (580) is configured to actively control the footwear configuration at least once per full operation cycle or locomotion stride, or many times per full operation cycle or locomotion stride, or once per many full operation cycles or locomotion strides, or based on a set time period of any duration or based on another test condition.

5. The smartphone or other mobile computer device (580) of any one of claims 1-4, wherein the smartphone or other mobile computer device (580) is configured to record a first test data set for a first configuration of the footwear and a second test data set for a second configuration of the footwear, each said data set consisting of measurements of the force and/or the relative pressure distribution of the wearer's footsole on an upper surface of the footwear during the wearer's locomotion or other physical activity, as measured in at least one plane (1D) or in two planes (2D) or in three planes (3D) and/or including time or other measurements, the footwear upper surface including at least a multitude or 20 or 50 or 100 or 500 or 1,000 or 4,000, or 16,000 individual pressure sensors (582);
compare the first test data set and the second test data set with a preferred data set for measurements of force and/or relative pressure distribution of the foot sole of a model wearer or wearers on an upper surface of the footwear during the locomotion or other physical activity, as measured in at least one plane (1D) or in two planes (2D) or in three planes (3D) and/or including time or other measurements; and
select the configuration setting of the soles that produced the test data set for the force or relative pressure distribution that is the closest to the preferred data set for relative pressure distribution and to reject the other configuration setting, thereby completing at least one full cycle of an operation to optimize the wearer's footwear configuration; and wherein the optimizing operation is used to reduce a range of pronation and/or supination of the wearer's foot and ankle during a landing phase of locomotion through active configuration of the either or both of the footwear soles or removable sole inserts.

6. The smartphone or other mobile computer device (580) of claim 5, configured to measure the relative positions to each other of a wearer's right and left feet during a stance phase of locomotion so as to determine a degree of crossover of right and/or left feet across a centerline of the wearer's body, as measured in a frontal plane during the stance phase of locomotion; and then to test a series of configuration settings in order to reduce or eliminate crossover.

7. The smartphone or other mobile computer device (580) of claim 1, configured to carry out the following steps:
first, control the footwear bladders, compartments, chambers or internal sipes by establishing a first configuration setting of said footwear bladders, compartments, chambers or internal sipes for at least a first test during locomotion or other physical activity of the wearer;
second, control the footwear bladders, compartments, chambers or internal sipes by establishing a second configuration setting of said footwear bladders, compartments, chambers or internal sipes that is different from the first configuration setting for at least a second test during locomotion or other physical activity of the wearer;
third, process measurement data from the first and second tests received from the sensors (582) located in both footwear soles or inserts and from the at least one sensor (582) in the smartphone or other mobile computer device (580) and/or in another device and/or located in the apparatus;
fourth, compare the data from the first and second tests with a preferred data set; and
fifth, select one said configuration setting of the footwear bladders, compartments, chambers or internal sipes from the first or second test that produced data that is closest to matching the preferred data set

8. The smartphone or other mobile computer device (580) of claim 1, configured to use the smartphone or other mobile computer device (580) to record and compare multiple test data sets consisting of measurements of relative motion during the wearer's locomotion or other physical activity of a position at or near to a part of the body of the wearer; as measured in at least one plane (1D) or in two planes (2D) or in three planes (3D) and/or including time or other measurements.

9. The smartphone or other mobile computer device (580) of claim 1, configured to use the smartphone or other mobile computer device (580) to record and compare multiple test data sets including data from at least the one sensor (582) of the smartphone or other mobile computer device (580) and consisting of measurements of relative motion during the wearer's locomotion or other physical activity of a position that is at or near a center of gravity (C.G.) of a body of the wearer, as measured in at least one plane (1D) or in two planes (2D) or in three planes (3D) and/or including time or other measurements.

10. The smartphone or other mobile computer device (580) of claim 1, wherein the smartphone or other mobile computer device (580) is configured to receive input from the apparatus with at least one gyroscope and/or accelerometer and/or other relative and/or absolute motion sensor (582) and/or pressure sensor (582) and/or force sensor (582), the apparatus comprising any article of clothing or personal equipment, including earphones or earplugs, helmet, eyeglasses, watch, belt, waistband, elastic underwear, armband, a taped or bandaged attachment, necklace, lanyard, cervical collar, ring, headband, body piercing, or that are in any manner attached or embedded in conventional or specialized clothing worn or attached to the skin of a wearer, or padding or braces, armor, or seating or furniture.

11. The smartphone or other mobile computer device (580) of claim 1, wherein the smartphone or other mobile computer device (580) is configured to control the apparatus worn or carried by the wearer, attached to the wearer, or implanted in the wearer,
the apparatus including one or more bladders, compartments, chambers or internal sipes that are configured for computer control so that the smartphone or other mobile computer device (580) alters the configuration of the one or more bladders, compartments, chambers or internal sipes of the apparatus.

12. The smartphone or other mobile computer device (580) of claim 1, wherein the smartphone or other mobile computer device (580) is configured to measure and receive 1D or 2D or 3D data of the relative motion of the wearer's head during locomotion using earplug sensor fixation by the ear canals of the wearer's head and compare the wearer's head motion data with the wearer's center of gravity (C.G.) data during locomotion or other physical activity; and/or
send either or both sets of data to a computer system and/or a third party for comparison and/or to conduct other functions in a shared operation, including a partially shared operation.

13. The smartphone or other mobile computer device (580) of any one of claims 1-12, wherein the smartphone or other mobile computer device (580) actively controls the configuration of the one or more bladders, compartments, chambers or internal sipes based on input from at least one sensor (582) located in the smartphone or other mobile computer device (580) and selected from a gyroscope and an accelerometer.

14. The smartphone or other mobile computer device (580) of any one of claims 1-12, wherein the smartphone or other mobile computer device (580) actively controls the configuration of the one or more bladders, compartments, chambers or internal sipes based on input from a gyroscope located in the smartphone or other mobile computer device (580) and an accelerometer located in the smartphone or other mobile computer device (580).

15. The smartphone or other mobile computer device (580) of any one of claims 1-14, wherein the smartphone or other mobile computer device (580) actively controls the configuration of the one or more bladders, compartments, chambers or internal sipes based on input from at least one sensor (582) located in the apparatus and selected from a gyroscope and an accelerometer.

16. The smartphone or other mobile computer device (580) of any one of claims 1-14, wherein the smartphone or other mobile computer device (580) actively controls the configuration of the one or more bladders, compartments, chambers or internal sipes based on input from a gyroscope located in the apparatus and an accelerometer located in the apparatus.

17. A computer system comprising a Web site and/or a cloud array of computers wherein the computer system is configured to have a connection to the smartphone or other mobile computer device (580) of any one of claims 1-16, **characterized in that**
the computer system is configured to use the smartphone or other mobile computer device (580) to actively control the configuration of the one or more bladders, compartments, chambers or internal sipes based on input from one or more sensors (582) located in either one or both of a sole or a removable sole insert of the footwear of the wearer and from at least one sensor (582) selected from a gyroscope and an accelerometer located in the smartphone or other mobile computer device (580) or in another device or in an apparatus worn or carried by the wearer, attached to the wearer, or implanted in the wearer.

18. The computer system of claim 17, wherein the computer system is further configured to share access by permitted third parties and by the wearer.

19. The computer system of claim 18, wherein the computer system is further configured for conducting real time or subsequent testing involving the one or more authorized third parties.

20. The computer system of claim 18, wherein the computer system is further configured to analyze test data sets of groups or categories of wearers.

21. A sole or a removable sole insert of footwear of a wearer wherein the sole or the removable sole insert is configured to have a connection to the smartphone or other mobile computer device (580) of any one of claims 1-16, **characterized in that**
the sole or the removable sole insert is configured to be controlled by the smartphone or other mobile computer device (580) in order for the smartphone or other mobile computer device (580) to actively control the configuration of the one or more bladders, compartments, chambers or internal sipes of the sole or the removable insert based on input from one or more sensors (582) located in either one or both of the sole or the removable sole insert of the footwear of the wearer and from at least one sensor (582) selected from a gyroscope and an accelerometer located in the smartphone or other mobile computer device (580) or in another device or in an apparatus worn or carried by the wearer, attached to the wearer, or implanted in the wearer, the apparatus or the other device being separate from the footwear of the wearer.

22. A computer program stored on a computer readable media, comprising:
a configuration for a smartphone or other mobile computer device (580) as claimed in any one of claims 1-16, whereby the smartphone or other mobile computer device (580) can actively control the configuration of one or more bladders, compartments, chambers or internal sipes located in either one or both of the sole or the removable sole insert of the footwear of the wearer and configured for computer control, **characterized in that**
the software, when executed on a smartphone or other mobile computer device (580), actively controls the configuration of the one or more bladders, compartments, chambers or internal sipes based on input from one or more sensors (582) located in either one or both of a sole or a removable sole insert of the footwear of the wearer and from at least one sensor (582) selected from a gyroscope and an accelerometer located in the smartphone or other mobile computer device (580) or in an apparatus worn or carried by the wearer, attached to the wearer, or implanted in the wearer, the apparatus or the other device being separate from the footwear of the wearer.

## Patentansprüche

1. Ein Smartphone oder eine andere mobile Computervorrichtung (580), universell oder spezialisiert, umfassend:
eine Konfiguration des Smartphones oder der anderen mobilen Computervorrichtung (580), wobei das Smartphone oder die andere mobile Computervorrichtung (580) die Konfiguration einer oder mehrerer Blasen, Fächer, Kammern oder inneren Lamellen, die sich entweder in einer oder beiden von einer Sohle oder einem herausnehmbaren Sohleneinsatz des Schuhwerks eines Trägers befinden, aktiv steuern kann, wobei die eine oder mehreren Blasen, Fächer, Kammern oder Lamellen zur Computersteuerung eingerichtet sind, **dadurch gekennzeichnet, dass**
das Smartphone oder die andere mobile Computervorrichtung (580) die Konfiguration der einen oder mehreren Blasen, Fächer, Kammern oder inneren Lamellen basierend auf der Eingabe von einem oder mehreren Messfühlern (582) aktiv steuert, die sich entweder in einem oder beiden von einer Sohle oder einem herausnehmbaren Sohleneinsatz des Schuhwerks des Trägers befinden, und von mindestens einem Messfühler (582), der aus einem Gyroskop und einem Beschleunigungsmesser ausgewählt ist, der sich in dem Smartphone oder der anderen mobilen Computervorrichtung (580) oder in einer anderen Vorrichtung oder in einer Einrichtung befindet, die von dem Träger getragen wird, an dem Träger befestigt oder in dem Träger implantiert ist, wobei die Vorrichtung oder andere Einrichtung von dem Schuhwerk des Trägers getrennt ist.

2. Smartphone oder andere mobile Computervorrichtung (580) nach Anspruch 1, wobei das Smartphone oder die andere mobile Computervorrichtung (580) die Konfiguration einer oder mehrerer Blasen, Fächer, Kammern oder inneren Lamellen aktiv steuern kann, basierend auf der Eingabe von einem oder mehreren Messfühlern (582), die sich entweder in einem oder beiden von einer Sohle oder einem herausnehmbaren Sohleneinsatz des Schuhwerks des Trägers und eines Gyroskops befinden, und von einem Beschleunigungsmesser, der unabhängig in dem Smartphone oder einer anderen mobilen Computervorrichtung (580) oder in der anderen Vorrichtung oder in der Einrichtung angeordnet ist.

3. Smartphone oder andere mobile Computervorrichtung (580) nach einem der Ansprüche 1 bis 2, wobei das Smartphone oder die andere mobile Computervorrichtung (580) eine magnetorheologische Flüssigkeit zum Teil oder vollständig aktiv steuern kann, die sich in der einen oder mehreren Blasen, Fächern, Kammern oder inneren Lamellen befindet, und/oder das Smartphone oder die andere mobile Computervorrichtung (580) mindestens ein Ventil zum Teil oder vollständig aktiv steuern kann, das sich zwischen zwei oder mehr der Blasen, Fächer, Kammern oder inneren Lamellen befindet, und/oder das Smartphone oder andere mobile Computervorrichtung (580) mindestens eine elektrische und/oder elektronische und/oder elektromechanische Vorrichtung zum Teil oder vollständig aktiv steuern kann.

4. Smartphone oder andere mobile Computervorrichtung (580) nach einem der Ansprüche 1 bis 3, wobei das Smartphone oder die andere mobile Computervorrichtung (580) eingerichtet ist, um die Konfiguration einer oder mehrerer Schuhsohlen oder herausnehmbarer Sohleneinsätze des Trägers aktiv zu steuern, indem es eine relative Längshöhe, samt positiver oder negativer Fersenanhebung, oder negativer oder positiver Vorfußanhebung und/oder einer relativen Seiten-Seiten-Höhe zwischen lateralen Seiten und mittleren Seiten und/oder einer relativen Höhe zwischen rechter und linker Schuhsohle oder herausnehmbaren Sohleneinsätzen oder einer Kombination dieser relativen Höhenänderungen verändert, und/oder
das Smartphone oder die andere mobile Computervorrichtung (580) eingerichtet ist, um die Konfiguration einer oder mehrerer Schuhsohlen oder herausnehmbarer Sohleneinsätze des Trägers aktiv zu steuern, indem es die relative Längsfestigkeit zwischen Fersenbereich und Vorfußbereich und/oder Seite-zu-Seite-Festigkeit zwischen lateralen und medialen Seitenbereichen und/oder die relative Festigkeit zwischen einer rechten und einer linken Schuhsohle oder einem herausnehmbaren Sohleneinsatz oder einer Kombination dieser relativen Festigkeitsänderungen verändert, und/oder
das Smartphone oder die andere mobile Computervorrichtung (580) eingerichtet ist, um die Konfiguration einer oder mehrerer Schuhsohlen oder herausnehmbarer Sohleneinlagen des Trägers aktiv zu steuern, indem es die relative Höhe oder Festigkeit unter einem oder mehreren der Fußknochen des Trägers, einschließlich unter einem Fersenbein, einem seitlichen Fersenbeinhöcker, einer Basis eines fünften Mittelfußknochens, jedem Kopf eines Mittelfußknochens und jedem distalen Phalangen, einschließlich eines Hallux oder einer großen Zehe, oder unter einem Längsbogen oder einem Mittelfußbogen des Trägers, verändert, und/oder
das Smartphone oder die andere mobile Computervorrichtung (580) eingerichtet ist, um die Konfiguration des Schuhwerks mindestens einmal pro vollem Betriebszyklus oder Fortbewegungsschritt oder mehrmals pro vollem Betriebszyklus oder Fortbewegungsschritt oder einmal pro viele volle Betriebszyklen oder Fortbewegungsschritte, oder basierend auf einer festgelegten Zeitspanne beliebiger Dauer oder basierend auf einer anderen Testbedingung, aktiv zu steuern.

5. Smartphone oder andere mobile Computervorrichtung (580) nach einem der Ansprüche 1 bis 4, wobei das Smartphone oder die andere mobile Computervorrichtung (580) eingerichtet ist, um einen ersten Testdatensatz für eine erste Konfiguration des Schuhwerks und einen zweiten Testdatensatz für eine zweite Konfiguration des Schuhwerks aufzuzeichnen, wobei jeder dieser Datensätze aus Messungen der Kraft und/oder der relativen Druckverteilung der Fußsohle des Trägers auf einer oberen Oberfläche des Schuhwerks während der Fortbewegung oder einer anderen körperlichen Aktivität des Trägers besteht, gemessen in mindestens einer Ebene (1D) oder in zwei Ebenen (2D) oder in drei Ebenen (3D) und/oder samt Zeit- oder anderen Messungen, wobei die obere Oberfläche des Schuhwerks mindestens eine Vielzahl von 20 oder 50 oder 100 oder 500 oder 1.000 oder 4.000 oder 16.000 einzelne Druckfühler (582) beinhaltet,
um den ersten Testdatensatz und den zweiten Testdatensatz mit einem bevorzugten Datensatz für Messungen der Kraft- und/oder relativen Druckverteilung der Fußsohle eines oder mehrerer Modellträger oder Träger auf eine obere Oberfläche des Schuhwerks während der Fortbewegung oder anderer körperlicher Aktivität zu vergleichen, gemessen in mindestens einer Ebene (1D) oder in zwei Ebenen (2D) oder in drei Ebenen (3D) und/oder samt Zeit- oder anderen Messungen, und
um die Konfigurationseinstellung der Sohlen auszuwählen, die den Testdatensatz für die Kraft- oder relative Druckverteilung erzeugt haben, die dem bevorzugten Datensatz für die relative Druckverteilung am nächsten liegt, und die andere Konfigurationseinstellung zu verwerfen, wodurch mindestens ein vollständiger Zyklus einer Operation abgeschlossen wird, um die Schuhwerkskonfiguration des Trägers zu optimieren, und wobei der Optimierungsvorgang verwendet wird, um einen Bereich der Pronation und/oder Supination von Fuß und Sprunggelenk des Trägers während einer Landephase der Fortbewegung durch aktive Konfiguration von einem oder beiden der Schuhsohlen oder der herausnehmbaren Sohleneinsätze zu reduzieren.

6. Smartphone oder andere mobile Computervorrichtung (580) nach Anspruch 5, die eingerichtet ist, um die relativen Positionen des rechten und des linken Fußes eines Trägers während einer Standphase der Fortbewegung zu messen, um einen Überkreuzungsgrad des rechten und/oder des linken Fußes über eine Mittellinie des Körpers des Trägers zu bestimmen, wie er in einer frontalen Ebene während der Haltungsphase der Fortbewegung gemessen wird, und dann um eine Reihe von Konfigurationseinstellungen zu testen, um eine Überkreuzung zu vermindern oder zu unterbinden.

7. Smartphone oder andere mobile Computervorrichtung (580) nach Anspruch 1, die eingerichtet ist, um die folgenden Schritte auszuführen:
erstens, Steuern der Blasen, Fächer, Kammern oder inneren Lamellen des Schuhwerks, indem eine erste Konfigurationseinstellung der Blasen, Fächer, Kammern oder inneren Lamellen des Schuhwerks für mindestens einen ersten Test während der Fortbewegung oder anderen körperlichen Aktivität des Trägers vorgenommen wird,
zweitens, Steuern der Blasen, Fächer, Kammern oder inneren Lamellen des Schuhwerks durch Herstellen einer zweiten Konfigurationseinstellung der Blasen, Fächer, Kammern oder inneren Lamellen des Schuhwerks, die sich von der ersten Konfigurationseinstellung für mindestens einen zweiten Test während der Fortbewegung oder anderen körperlichen Aktivität des Trägers unterscheidet,
drittens, Verarbeiten von Messdaten aus dem ersten und dem zweiten Test, die von den Messfühlern (582) empfangen werden, die sich sowohl in Schuhsohlen oder Einsätzen als auch von dem mindestens einen Messfühler (582) in dem Smartphone oder einer anderen mobilen Computervorrichtung (580) und/oder in einer anderen Vorrichtung und/oder in der Einrichtung befinden,
viertens, Vergleichen der Daten aus dem ersten und dem zweiten Test mit einem bevorzugten Datensatz, und
fünftens, Wählen einer der Konfigurationseinstellungen der Blasen, Fächer, Kammern oder inneren Lamellen des Schuhwerks aus dem ersten oder dem zweiten Test, der Daten erzeugt hat, die dem bevorzugten Datensatz am nächsten kommen.

8. Smartphone oder andere mobile Computervorrichtung (580) nach Anspruch 1, die eingerichtet ist, um das Smartphone oder die andere mobile Computervorrichtung (580) zu verwenden, um mehrere Testdatensätze aufzuzeichnen und zu vergleichen, die aus Messungen der Relativbewegung während der Fortbewegung des Trägers oder einer anderen körperlichen Aktivität einer Position an oder nahe an einem Teil des Körpers des Trägers bestehen, gemessen in mindestens einer Ebene (1D) oder in zwei Ebenen (2D) oder in drei Ebenen (3D) und/oder samt Zeit- oder anderen Messungen.

9. Smartphone oder andere mobile Computervorrichtung (580) nach Anspruch 1, die eingerichtet ist, um das Smartphone oder die andere mobile Computervorrichtung (580) zu verwenden, um mehrere Testdatensätze aufzuzeichnen und zu vergleichen, die Daten von mindestens dem einen Messfühler (582) des Smartphones oder der anderen mobilen Computervorrichtung (580) beinhalten und aus Messungen der Relativbewegung während der Fortbewegung des Trägers oder einer anderen körperlichen Aktivität einer Position bestehen, die sich in oder in der Nähe eines Schwerpunktes (C.G.) eines Körpers des Trägers befindet, gemessen in mindestens einer Ebene (1D) oder in zwei Ebenen (2D) oder in drei Ebenen (3D).

10. Smartphone oder andere mobile Computervorrichtung (580) nach Anspruch 1, wobei das Smartphone oder die andere mobile Computervorrichtung (580) eingerichtet ist, um Eingaben von der Vorrichtung mit mindestens einem Gyroskop und/oder Beschleunigungsmesser und/oder einem anderen relativen und/oder absoluten Bewegungsmesser (582) und/oder Druckfühler (582) und/oder Kraftsensor (582) zu empfangen, wobei die Vorrichtung jedes beliebige Kleidungsstück oder jede beliebige persönliche Ausrüstung wie Kopfhörer oder Ohrstöpsel, Helm, Brille, Uhr, Gürtel, Bund, elastische Unterwäsche, Armband, geklebte oder bandagierte Befestigung, Halskette, Umhängeband, Halskragen, Ring, Stirnband, Körper-Piercing oder solche umfasst, die in beliebiger Weise in konventioneller oder spezieller Kleidung, die getragen oder an der Haut eines Trägers befestigt ist, oder Polsterung oder Hosenträger, Rüstung oder Sitz oder Möbel befestigt sind oder in diese eingebettet sind.

11. Smartphone oder andere mobile Computervorrichtung (580) nach Anspruch 1, wobei das Smartphone oder die andere mobile Computervorrichtung (580) eingerichtet ist, um die Vorrichtung zu steuern, die von dem Träger getragen wird, an dem Träger befestigt oder in den Träger implantiert ist,
die Vorrichtung eine oder mehrere Blasen, Fächer, Kammern oder innere Lamellen umfasst, die zur Computersteuerung eingerichtet sind, so dass das Smartphone oder die andere mobile Computervorrichtung (580) die Konfiguration der einen oder mehreren Blasen, Fächer, Kammern oder inneren Lamellen der Vorrichtung verändert.

12. Smartphone oder andere mobile Computervorrichtung (580) nach Anspruch 1, wobei das Smartphone oder die andere mobile Computervorrichtung (580) eingerichtet ist, um 1D- oder 2D- oder 3D-Daten der Relativbewegung des Kopfes des Trägers während der Fortbewegung unter Verwendung einer Ohrstöpsel-Messfühlerfixierung durch die Gehörgänge des Kopfes des Trägers zu messen und zu empfangen und die Kopfbewegungsdaten des Trägers mit den Daten des Schwerpunktes (C.G.) des Trägers während der Fortbewegung oder anderer körperlicher Aktivität zu vergleichen, und/oder
Senden eines oder beider Datensätze an ein Computersystem und/oder einen Dritten zum Vergleich und/oder zur Durchführung anderer Funktionen in einer gemeinsamen Operation, einschließlich einer partiell gemeinsamen Operation.

13. Smartphone oder andere mobile Computervorrichtung (580) nach einem der Ansprüche 1 bis 12, wobei das Smartphone oder die andere mobile Computervorrichtung (580) die Konfiguration der einen oder mehreren Blasen, Fächer, Kammern oder inneren Lamellen aktiv steuert, basierend auf der Eingabe von mindestens einem Messfühler (582), der sich in dem Smartphone oder der anderen mobilen Computervorrichtung (580) befindet und ausgewählt ist aus einem Gyroskop und einem Beschleunigungsmesser.

14. Smartphone oder andere mobile Computervorrichtung (580) nach einem der Ansprüche 1 bis 12, wobei das Smartphone oder die andere mobile Computervorrichtung (580) die Konfiguration der einen oder mehreren Blasen, Fächer, Kammern oder inneren Lamellen aktiv steuert, basierend auf der Eingabe von mindestens einem Gyroskop, das sich in dem Smartphone oder der anderen mobilen Computervorrichtung (580) befindet, und von einem Beschleunigungsmesser, der sich in dem Smartphone oder der anderen mobilen Computervorrichtung (580) befindet.

15. Smartphone oder andere mobile Computervorrichtung (580) nach einem der Ansprüche 1 bis 14, wobei das Smartphone oder die andere mobile Computervorrichtung (580) die Konfiguration der einen oder mehreren Blasen, Fächer, Kammern oder inneren Lamellen aktiv steuert, basierend auf der Eingabe von mindestens einem Messfühler (582), der sich in der Vorrichtung befindet und ausgewählt ist aus einem Gyroskop und einem Beschleunigungsmesser.

16. Smartphone oder andere mobile Computervorrichtung (580) nach einem der Ansprüche 1 bis 14, wobei das Smartphone oder die andere mobile Computervorrichtung (580) die Konfiguration der einen oder mehreren Blasen, Fächer, Kammern oder inneren Lamellen aktiv steuert, basierend auf der Eingabe von einem in der Vorrichtung befindlichen Gyroskop und einem in der Vorrichtung befindlichen Beschleunigungsmesser.

17. Computersystem, umfassend eine Website und/oder eine Cloud-Anordnung von Computern, wobei das Computersystem eingerichtet ist, um eine Verbindung mit dem Smartphone oder der anderen mobilen Computervorrichtung (580) nach einem der Ansprüche 1 bis 16 zu haben, **dadurch gekennzeichnet, dass**
das Computersystem eingerichtet ist, um das Smartphone oder die andere mobile Computervorrichtung (580) zu verwenden, um die Konfiguration der einen oder mehreren Blasen, Fächer, Kammern oder inneren Lamellen basierend auf der Eingabe von einem oder mehreren Messfühlern (582) aktiv zu steuern, die sich entweder in einer oder beiden von einer Sohle oder einem herausnehmbaren Sohleneinsatz des Schuhwerks des Trägers befinden, und von mindestens einem Messfühler (582), der aus einem (Gyroskop und einem Beschleunigungsmesser ausgewählt ist, der sich in dem Smartphone oder der anderen mobilen Computervorrichtung (580) oder in einer anderen Vorrichtung oder in einer Einrichtung befindet, die von dem Träger getragen wird, an dem Träger befestigt oder in dem Träger implantiert ist.

18. Computersystem nach Anspruch 17, wobei das Computersystem ferner eingerichtet ist, um den Zugriff durch zugelassene Dritte und durch den Träger gemeinsam zu nutzen.

19. Computersystem nach Anspruch 18, wobei das Computersystem ferner eingerichtet ist, um Echtzeit- oder Folgetests unter Beteiligung eines oder mehrerer autorisierter Dritter vorzunehmen.

20. Computersystem nach Anspruch 18, wobei das Computersystem ferner eingerichtet ist, um Testdatensätze von Gruppen oder Kategorien von Trägern zu analysieren.

21. Sohle oder eine herausnehmbare Sohle im Schuhwerk eines Trägers, wobei die Sohle oder der herausnehmbare Sohleneinsatz eingerichtet ist, um eine Verbindung mit dem Smartphone oder einer anderen mobilen Computervorrichtung (580) nach einem der Ansprüche 1 bis 16 zu haben, **dadurch gekennzeichnet, dass**
die Sohle oder der herausnehmbare Sohleneinsatz eingerichtet ist, um durch das Smartphone oder die andere mobile Computervorrichtung (580) gesteuert zu werden, damit das Smartphone oder die andere mobile Computervorrichtung (580) die Konfiguration der einen oder mehreren Blasen, Fächer, Kammern oder inneren Lamellen der Sohle oder des herausnehmbaren Einsatzes aktiv steuert, basierend auf der Eingabe von einem oder mehreren Messfühlern (582), die sich entweder in einer oder beiden von Sohle oder herausnehmbarem Sohleneinsatz des Schuhwerks des Trägers befinden, und von mindestens einem Messfühler (582), der aus einem Gyroskop und einem Beschleunigungsmesser ausgewählt ist, der sich in dem Smartphone oder der anderen mobilen Computervorrichtung (580) oder in einer anderen Vorrichtung oder in einer Einrichtung befindet, die vom Träger getragen wird, an dem Träger befestigt oder in den Träger implantiert ist, wobei die Vorrichtung oder die andere Einrichtung vom Schuhwerk des Trägers getrennt ist.

22. Auf einem computerlesbaren Medium gespeichertes Computerprogramm, umfassend:
eine Konfiguration für ein Smartphone oder eine andere mobile Computervorrichtung (580), wie in einem der Ansprüche 1 bis 16 beansprucht, wobei das Smartphone oder die andere mobile Computervorrichtung (580) die Konfiguration einer oder mehrerer Blasen, Fächer, Kammern oder inneren Lamellen, die sich entweder in einer oder beiden von Sohle oder herausnehmbarem Sohleneinsatz des Schuhwerks des Trägers befinden und für die Computersteuerung eingerichtet sind, aktiv steuern kann, **dadurch gekennzeichnet, dass**
die Software, wenn sie auf einem Smartphone oder einer anderen mobilen Computervorrichtung (580) ausgeführt wird, die Konfiguration der einen oder mehreren Blasen, Fächer, Kammern oder inneren Lamellen basierend auf der Eingabe von einem oder mehreren Messfühlern (582) aktiv steuert, die sich entweder in einem oder beiden einer Sohle oder eines herausnehmbaren Sohleneinsatzes des Schuhwerks des Trägers befinden, und von mindestens einem Messfühler (582), der aus einem Gyroskop und einem Beschleunigungsmesser ausgewählt ist, der sich in dem Smartphone oder der anderen mobilen Computervorrichtung (580) oder in einer Vorrichtung befindet, die vom Träger getragen wird, an dem Träger befestigt oder in den Träger implantiert ist, wobei die Vorrichtung oder die andere Einrichtung vom Schuhwerk des Trägers getrennt ist.

## Revendications

1. Téléphone intelligent ou autre dispositif informatique mobile (580), polyvalent ou spécialisé, comprenant :
une configuration du téléphone intelligent ou autre dispositif informatique mobile (580) par laquelle le téléphone intelligent ou autre dispositif informatique mobile (580) peut commander activement la configuration d'un ou plusieurs compartiments, vessies, chambres ou lamelles internes situés dans l'un, l'autre ou les deux d'une semelle ou d'un insert de semelle amovible de la chaussure d'un porteur, le ou les compartiments, vessies, chambres ou lamelles internes étant configurés pour être commandés par informatique, **caractérisé en ce que**
le téléphone intelligent ou autre dispositif informatique mobile (580) commande activement la configuration du ou des compartiments, vessies, chambres ou lamelles internes sur la base d'une entrée d'un ou plusieurs capteurs (582) situés dans l'un, l'autre ou les deux d'une semelle ou d'un insert de semelle amovible de la chaussure du porteur et d'au moins un capteur (582) choisi parmi un gyroscope et un accéléromètre situé dans le téléphone intelligent ou autre dispositif informatique mobile (580) ou dans un autre dispositif ou dans un appareil porté ou transporté par le porteur, fixé au porteur, ou implanté dans le porteur, l'appareil ou l'autre dispositif étant distinct de la chaussure du porteur.

2. Téléphone intelligent ou autre dispositif informatique mobile (580) selon la revendication 1, le téléphone intelligent ou autre dispositif informatique mobile (580) pouvant commander activement la configuration d'un ou plusieurs compartiments, vessies, chambres ou lamelles internes sur la base d'une entrée d'un ou plusieurs capteurs (582) situés dans l'un, l'autre ou les deux d'une semelle ou d'un insert de semelle amovible de la chaussure du porteur et d'un gyroscope et d'un accéléromètre situés indépendamment dans le téléphone intelligent ou autre dispositif informatique mobile (580) ou dans l'autre dispositif ou dans l'appareil.

3. Téléphone intelligent ou autre dispositif informatique mobile (580) selon l'une quelconque des revendications 1 à 2, le téléphone intelligent ou autre dispositif informatique mobile (580) pouvant commander activement, en partie ou complètement, un fluide magnéto-rhéologique situé dans le ou les compartiments, vessies, chambres ou lamelles internes ; et/ou le téléphone intelligent ou autre dispositif informatique mobile (580) pouvant commander activement, en partie ou complètement, au moins une vanne située entre deux ou plusieurs desdits compartiments, vessies, chambres ou lamelles internes ; et/ou le téléphone intelligent ou autre dispositif informatique mobile (580) pouvant commander activement, en partie ou complètement, au moins un dispositif électrique et/ou électronique et/ou électromécanique.

4. Téléphone intelligent ou autre dispositif informatique mobile (580) selon l'une quelconque des revendications 1 à 3, le téléphone intelligent ou autre dispositif informatique mobile (580) étant configuré pour commander activement la configuration d'une ou plusieurs semelles de chaussure ou inserts de semelle amovibles du porteur en modifiant une hauteur longitudinale relative, y compris une élévation positive ou négative du talon, ou une élévation négative ou positive de l'avant-pied, et/ou une hauteur relative de côté à côté entre des côtés latéraux et médians, et/ou une hauteur relative entre des semelles de chaussure ou des inserts de semelle amovible droit et gauche, ou une combinaison de ces modifications de hauteur relative ; et/ou le téléphone intelligent ou autre dispositif informatique mobile (580) étant configuré pour commander activement la configuration d'une ou plusieurs semelles de chaussure ou inserts de semelle amovibles du porteur en modifiant la fermeté longitudinale relative entre la zone de talon et la zone d'avant-pied et/ou la fermeté de côté à côté entre des zones de côté latérales et médianes, et/ou une fermeté relative entre une semelle de chaussure ou un insert de semelle amovible droit et gauche, ou une combinaison de ces modifications de fermeté relatives ; et/ou
le téléphone intelligent ou autre dispositif informatique mobile (580) est configuré pour commander activement la configuration d'une ou plusieurs semelles de chaussure ou inserts de semelle amovibles du porteur en modifiant la fermeté ou la hauteur relative sous l'un des os du pied du porteur, y compris sous un calcaneus, une tubérosité calcanéenne latérale, une base du cinquième métatarse, chaque tête de métatarse et chaque phalange distale, y compris un hallux ou gros orteil, ou sous une voute longitudinale ou une voute métatarsienne du porteur ; et/ou
le téléphone intelligent ou autre dispositif informatique mobile (580) étant configuré pour commander activement la configuration de la chaussure au moins une fois par cycle opératoire complet ou foulée de locomotion, ou plusieurs fois par cycle opératoire complet ou foulée de locomotion, ou une fois pour plusieurs cycles opératoires complets ou foulées de locomotion, ou sur la base d'un laps de temps défini d'une quelconque durée ou sur la base d'une autre condition d'essai.

5. Téléphone intelligent ou autre dispositif informatique mobile (580) selon l'une quelconque des revendications 1 à 4, le téléphone intelligent ou autre dispositif informatique mobile (580) étant configuré pour enregistrer un premier ensemble de données d'essai pour une première configuration de la chaussure et un second ensemble de données d'essai pour une seconde configuration de la chaussure, chaque ensemble de données étant constitué de mesures de force et/ou de distribution de la pression relative de la région plantaire du porteur sur une surface supérieure de la chaussure pendant la locomotion du porteur ou une autre activité physique, ainsi que mesurée sur au moins un plan (1D) ou sur deux plans (2D) ou sur trois plans (3D) et/ou incluant des mesures de temps et autre, la surface supérieure de la chaussure incluant au moins une multitude ou 20 ou 50 ou 100 ou 500 ou 1000 ou 4000 ou 16 000 capteurs de pression (582) individuels ;
comparer le premier ensemble de données d'essai et le second ensemble de données d'essai à un ensemble de données préféré pour la force et/ou la distribution de pression relative de la région plantaire d'un porteur ou de porteurs modèles sur une surface supérieure de la chaussure pendant la locomotion ou autre activité physique, ainsi que mesurée sur au moins un plan (1D) ou sur deux plans (2D) ou sur trois plans (3D) et/ou incluant des mesures de temps ou autre ; et
sélectionner le réglage de configuration des semelles qui a produit l'ensemble de données d'essai de force ou de distribution de pression relative qui est le plus proche de l'ensemble de données d'essai préféré pour la distribution de pression relative et rejeter l'autre réglage de configuration, ce qui permet de terminer au moins un cycle opératoire complet afin d'optimiser la configuration de la chaussure du porteur ; et l'opération d'optimisation étant utilisée pour réduire une plage de pronation et/ou de supination du pied et de la cheville du porteur pendant une phase de posé de la locomotion par le biais d'une configuration active de l'un, l'autre ou des deux parmi des semelles de chaussure ou inserts de semelle amovibles.

6. Téléphone intelligent ou autre dispositif informatique mobile (580) selon la revendication 5, configuré pour mesurer les positions relatives, l'un par rapport à l'autre, des pieds gauche et droit d'un porteur pendant une phase de levé de la locomotion de façon à déterminer un degré de croisement des pieds droit et/ou gauche à travers une ligne centrale du corps du porteur, ainsi que mesuré dans un plan frontal pendant la phase de levé de la locomotion ; puis tester une série de réglages de configuration afin de réduire ou d'éliminer le croisement.

7. Téléphone intelligent ou autre dispositif informatique mobile (580) selon la revendication 1, configuré pour exécuter les étapes suivantes :
premièrement, commander les compartiments, vessies, chambres ou lamelles internes de la chaussure en établissant un premier réglage de configuration desdits compartiments, vessies, chambres ou lamelles internes de chaussure pendant au moins un premier essai pendant une locomotion ou une autre activité physique du porteur ;
deuxièmement, commander les compartiments, vessies, chambres ou lamelles internes de la chaussure en établissant un second réglage de configuration desdits compartiments, vessies, chambres ou lamelles internes de chaussure qui est différent du premier réglage de configuration pendant au moins un second essai pendant la locomotion ou une autre activité physique du porteur ;
troisièmement, traiter les données de mesure des premier et second essais reçues des capteurs (582) situés dans les deux semelles ou inserts de chaussure et de l'au moins un capteur (582) dans le téléphone intelligent ou autre dispositif informatique mobile (580) et/ou dans un autre dispositif et/ou situé dans l'appareil ;
quatrièmement, comparer les données des premier et second essais à un ensemble de données préféré ; et
cinquièmement, sélectionner le réglage de configuration des compartiments, vessies, chambres ou lamelles internes de chaussure parmi le premier ou le second essai qui a produit les données qui correspondent au plus près à l'ensemble de données préféré

8. Téléphone intelligent ou autre dispositif informatique mobile (580) selon la revendication 1, configuré pour utiliser le téléphone intelligent ou autre dispositif informatique mobile (580) pour enregistrer et comparer plusieurs ensembles de données d'essai constitués de mesures de mouvement relatif, pendant la locomotion du porteur ou autre activité physique, d'une position au niveau ou à proximité d'une partie du corps du porteur ; ainsi que mesurées dans au moins un plan (1D) ou dans deux plans (2D) ou dans trois plans (3D) et/ou incluant des mesures de temps ou autre.

9. Téléphone intelligent ou autre dispositif informatique mobile (580) selon la revendication 1, configuré pour utiliser le téléphone intelligent ou autre dispositif informatique mobile (580) pour enregistrer et comparer plusieurs ensembles de données d'essai incluant des données d'au moins le capteur (582) du téléphone intelligent ou autre dispositif informatique mobile (580) et constitués de mesures de mouvement relatif, pendant la locomotion du porteur ou autre activité physique, d'une position au niveau ou à proximité d'un centre de gravité (C.G.) du corps du porteur, ainsi que mesurées dans au moins un plan (1D) ou dans deux plans (2D) ou dans trois plans (3D) et/ou incluant des mesures de temps ou autre.

10. Téléphone intelligent ou autre dispositif informatique mobile (580) selon la revendication 1, le téléphone intelligent ou autre dispositif informatique mobile (580) étant configuré pour recevoir une entrée de l'appareil avec au moins un gyroscope et/ou accéléromètre et/ou autre capteur de mouvement relatif et/ou absolu (582) et/ou capteur de pression (582) et/ou capteur de force (582), l'appareil comprenant tout article vestimentaire ou d'équipement personnel, y compris écouteurs ou bouchons d'oreille, casque, lunettes, montre, ceinture, sangle ventrale, sous-vêtement élastique, brassard, accessoire fixé par ruban ou bande, collier, lanière, tour de cou, bague, bandeau serre-tête, piercing corporel ou qui soit fixé ou intégré d'une quelconque manière dans des vêtements classiques ou spécialisés portés ou fixés sur la peau du porteur, ou un matelassage ou des bretelles, un élément de protection ou un siège ou un meuble.

11. Téléphone intelligent ou autre dispositif informatique mobile (580) selon la revendication 1, le téléphone intelligent ou autre dispositif informatique mobile (580) étant configuré pour commander l'appareil porté ou transporté par le porteur, fixé au porteur ou implanté dans le porteur,
le dispositif incluant une ou plusieurs compartiments, vessies, chambres ou lamelles internes qui sont configurés pour être commandés par informatique de sorte que le téléphone intelligent ou autre dispositif informatique mobile (580) modifie la configuration du ou des compartiments, vessies, chambres ou lamelles internes de l'appareil.

12. Téléphone intelligent ou autre dispositif informatique mobile (580) selon la revendication 1, le téléphone intelligent ou autre dispositif informatique mobile (580) étant configuré pour mesurer et recevoir des données 1D, 2D ou 3D du mouvement relatif de la tête du porteur pendant la locomotion au moyen d'une fixation de capteur de bouchon d'oreille par les canaux auditifs de la tête du porteur et comparer les données de mouvement de la tête du porteur aux données de centre de gravité (C.G.) du porteur pendant la locomotion ou autre activité physique ; et/ou
envoyer l'un, l'autre ou les deux ensembles de données à un système informatique et/ou une tierce partie à des fins de comparaison et/ou pour effectuer d'autres fonctions lors d'une opération partagée, y compris une opération partiellement partagée.

13. Téléphone intelligent ou autre dispositif informatique mobile (580) selon l'une quelconque des revendications 1 à 12, le téléphone intelligent ou autre dispositif informatique mobile (580) commandant activement la configuration du ou des compartiments, vessies, chambres ou lamelles internes sur la base d'une entrée d'au moins un capteur (582) situé dans le téléphone intelligent ou autre dispositif informatique mobile (580) et choisi parmi un gyroscope et un accéléromètre.

14. Téléphone intelligent ou autre dispositif informatique mobile (580) selon l'une quelconque des revendications 1 à 12, le téléphone intelligent ou autre dispositif informatique mobile (580) commandant activement la configuration du ou des compartiments, vessies, chambres ou lamelles internes sur la base d'une entrée d'un gyroscope situé dans le téléphone intelligent ou autre dispositif informatique mobile (580) et d'un accéléromètre situé dans le téléphone intelligent ou autre dispositif informatique mobile (580).

15. Téléphone intelligent ou autre dispositif informatique mobile (580) selon l'une quelconque des revendications 1 à 14, le téléphone intelligent ou autre dispositif informatique mobile (580) commandant activement la configuration du ou des compartiments, vessies, chambres ou lamelles internes sur la base d'une entrée d'au moins un capteur (582) situé dans l'appareil et choisi parmi un gyroscope et un accéléromètre.

16. Téléphone intelligent ou autre dispositif informatique mobile (580) selon l'une quelconque des revendications 1 à 14, le téléphone intelligent ou autre dispositif informatique mobile (580) commandant activement la configuration du ou des compartiments, vessies, chambres ou lamelles internes sur la base d'une entrée d'un gyroscope situé dans l'appareil et d'un accéléromètre situé dans l'appareil.

17. Système informatique comprenant un site Web et/ou un réseau d'ordinateurs dans le cloud, le système informatique étant configuré pour disposer d'une connexion au téléphone intelligent ou autre dispositif informatique mobile (580) selon l'une quelconque des revendications 1 à 16, **caractérisé en ce que**
le système informatique est configuré pour utiliser le téléphone intelligent ou autre dispositif informatique mobile (580) pour commander activement la configuration du ou des compartiments, vessies, chambres ou lamelles internes sur la base d'une entrée d'un ou plusieurs capteurs (582) situés dans l'un, l'autre ou les deux d'une semelle et d'un insert de semelle amovible de la chaussure du porteur et d'au moins un capteur (582) choisi parmi un gyroscope et un accéléromètre situé dans le téléphone intelligent ou autre dispositif informatique mobile (580) ou dans un autre dispositif ou dans un appareil porté ou transporté par le porteur, fixé au porteur, ou implanté dans le porteur.

18. Système informatique selon la revendication 17, le système informatique étant en outre configuré pour donner un accès partagé à des tierces parties autorisées et au porteur.

19. Système informatique selon la revendication 18, le système informatique étant en outre configuré pour effectuer des essais en temps réel ou ultérieurement impliquant la ou les tierces parties autorisées.

20. Système informatique selon la revendication 18, le système informatique étant en outre configuré pour analyser des ensembles de données d'essai de groupes ou de catégories de porteurs.

21. Semelle ou insert de semelle amovible de chaussure d'un porteur, la semelle ou l'insert de semelle amovible étant configuré pour disposer d'une connexion au téléphone intelligent ou autre dispositif informatique mobile (580) selon l'une quelconque des revendications 1 à 16, **caractérisé en ce que**
la semelle ou l'insert de semelle amovible est configuré pour être commandé par le téléphone intelligent ou autre dispositif informatique mobile (580) afin que le téléphone intelligent ou autre dispositif informatique mobile (580) puisse commander activement la configuration du ou des compartiments, vessies, chambres ou lamelles internes de la semelle ou de l'insert amovible
sur la base d'une entrée d'un ou plusieurs capteurs (582) situés dans l'un, l'autre ou les deux de la semelle ou de l'insert de semelle amovible de la chaussure du porteur et d'au moins un capteur (582) choisi parmi un gyroscope et un accéléromètre situé dans le téléphone intelligent ou autre dispositif informatique mobile (580) ou dans un autre dispositif ou dans un appareil porté ou transporté par le porteur, fixé au porteur, ou implanté dans le porteur, l'appareil ou l'autre dispositif étant distinct de la chaussure du porteur.

22. Programme informatique stocké sur un support lisible par un ordinateur, comprenant :
une configuration pour un téléphone intelligent ou autre dispositif informatique mobile (580) ainsi que revendiqué dans l'une quelconque des revendications 1 à 16, ce par quoi le téléphone intelligent ou autre dispositif informatique mobile (580) peut commander activement la configuration du ou des compartiments, vessies, chambres ou lamelles internes situés dans l'un, l'autre ou les deux de la semelle ou de l'insert de semelle amovible de la chaussure du porteur et configurés pour être commandés par informatique, **caractérisé en ce que**
le logiciel, quand il est exécuté sur un téléphone intelligent ou autre dispositif informatique mobile (580), commande activement la configuration du ou des compartiments, vessies, chambres ou lamelles internes sur la base d'une entrée d'un ou plusieurs capteurs (582) situés dans l'un, l'autre ou les deux d'une semelle ou d'un insert de semelle amovible de la chaussure du porteur et d'au moins un capteur (582) choisi parmi un gyroscope et un accéléromètre situé dans le téléphone intelligent ou autre dispositif informatique mobile (580) ou dans un appareil porté ou transporté par le porteur, fixé au porteur, ou implanté dans le porteur, l'appareil ou l'autre dispositif étant distinct de la chaussure du porteur.
